(19) Europäisches Patentamt
European Patent Office
Office européen des brevets

(11) **EP 2 977 077 A1**

(12) **EUROPEAN PATENT APPLICATION**

(43) Date of publication:
**27.01.2016 Bulletin 2016/04**

(51) Int Cl.:
**A61N 1/05** *(2006.01)*    **A61N 1/40** *(2006.01)*

(21) Application number: **15161397.3**

(22) Date of filing: **27.03.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **21.07.2014 US 201462027116 P**

(71) Applicants:
• **Lara Pereira Monteiro, Sergio**
  **Los Angeles, CA 90025 (US)**

• **Lee, Chong Il**
  **Stanton, CA 90680 (US)**

(72) Inventors:
• **Lara Pereira Monteiro, Sergio**
  **Los Angeles, CA 90025 (US)**
• **Lee, Chong Il**
  **Stanton, CA 90680 (US)**

(74) Representative: **Straus, Alexander**
  **Patentanwälte**
  **Becker, Kurig, Straus**
  **Bavariastrasse 7**
  **80336 München (DE)**

(54) **IMPLANTABLE ELECTRICAL STIMULATING DEVICE WITH ELECTRODES AND WITH SUPERCAPACITORS FOR ELECTRIC FIELD SHAPING**

(57)    An implantable electrical stimulating device (110, 124, 132, 140-t1, 140-t2, BAT1, MP1) is disclosed comprising an electric energy storage unit (BAT1), controlling electronics (MP1), a supporting structure (132), active electrodes (140-t1) and supercapacitors (140-t2) for shaping an electric field. The device is suitable for the heart, brain, other organs and general cells. The supercapacitor ensures a high stored charge and a high electric field magnitude.

*FIG. 2*

1 mm

**Description**

CROSS-REFERENCE TO RELATED APPLICATIONS

This application claims priority to US provisional patent application number 62 / 027,116 date 21 July 2014, entitled "Cell electric stimulator with electrodes for electrical field shaping and separate electrodes for stimulation", with the same inventors as this patent application.
This application is related to US patent number 8,954,145 date 10 Feb 2015, and also related to European patent application number EP 2012 0167688.6 application date 11 May 2012, publication number EP2522389 A3 on August 27 2014, with the same inventors as this patent application. We incorporate here, by reference, the full text, figures and claims of all these provisional and regular applications.

FEDERALLY SPONSORED RESEARCH

Not applicable

SEQUENCE LISTING OR PROGRAM

Not applicable

BACKGROUND OF THE INVENTION - field of Invention

This invention relates to electrical stimulation of cells in animals and other living forms, particularly to electrical stimulation of heart cells, including heart muscles associated with heart muscle contraction and with the His bundle, the left and right bundles, the Purkinje and similar fibers. The invention is applicable to artificial heart pacemakers. More precisely, the invention relates to causing an efficient contraction sequence of the heart muscle in order to maximize the volume of blood pumped per unit of energy spent by the heart. It also relates to the field of electrical stimulation of the cochlea, as in cochlear implants. It also relates to the field of electrical stimulation of neurons as in brain and peripheral neurons. Brain neurons are stimulated both for clinical objectives, as in Parkinson's disease control, and in animal research as well, in which case neurons are stimulated to observe the consequences of the stimulation. Other neurons are stimulated to block pain sensation on its way to the brain or other information transmitting function. An example of the latter is the information to the brain about the blood pressure in arteries and veins, as discussed in Dennis T. T. Plachta et al. [Plachta2014]. It also relates to the field of electrical stimulation of organs, as stomach, etc.

BACKGROUND - Discussion of existing devices and known pertinent facts

The phenomenon of muscle contraction and its electrical nature was first observed in the waning years of the 1700s by the great Italian Luigi Galvani (born 1737, dec. 1798), from Bologna, who noticed that a frog's leg contracted when subjected to an electric current. Today it is known that all our muscles, from a blinking eye to a walking leg and fingers pressing the keyboard of a computer to write the background section of a patent application, they all work on the same principles observed by Galvani - including out heart. The heart contracts as response to an electric pulse, which is injected on it at the required frequency, which varies according to the person's activity and state of excitation.
Broken to their building blocks, existing heart pacemakers are one or more electrodes 140, which is a fancy name for a tip of exposed metal, an electric battery and a controlling electronic circuit capable of generating pulses at the heart beat frequency of approximately 70 per minute, or a little less than one second each. The electrode is anchored in the heart, the battery and the controlling electronic circuit are located in a sealed box, usually just below the skin, in the chest of abdomen, with a connecting wire from the battery / controlling electronics to the electrode in the heart. The battery / controlling electronics are located in a sealed box **110** is of the approximate size as an ordinary cell phone, but with a much simpler electronics controlling unit, though, for some mysterious reason that escapes me the heart device costs one hundred times more than a cell phone. As it is known to the persons familiar with the electronics fabrication units, the cost disparity is not due to the need to keep the heart pacemaker clean of germs, because the electronics fabrication units are far cleaner than any surgery room. The electronic circuit is capable of creating an electric pulse at some periodicity, and capable of injecting a certain current in the region surrounding the electrode in the heart. The electrode itself is implanted in the heart, usually via a simple procedure involving inserting a wire with the electrode at its tip from a vein just below the clavicle (the sub-clavian vein), or some other convenient blood vessel, feeding the wire in while watching on an X-ray machine until the electrode reaches the heart, then anchoring the electrode into the inner wall of the heart. Variations of this basic design involve pacing-on-demand, which means pacing only when the natural mechanism fails, or a double or even triple electrode, and many other bells and whistles.

It is crucial here to keep in mind the difference between the electric current in metals (as in wires) and the electric current through the cells of an animal, as a human. The electric current in metals propagates by the motion of electrons, which are light particles moving through a mostly unopposed medium of the metal known as the conduction band; the electric current in wires go around the equator 5 times in a second (2/3 of the speed of light). The electric current in animal cells propagates by the motion of heavy ions (usually K, Na or Ca) in a difficult path to negotiate, suffering many collisions, besides dragging charges of the opposite polarity inside the cells, which increase their effective masses; they go the 10 cm length of the heart in 1 second - 2 billion times slower than electrons in wires. It is the slow speed (and longer propagation time) that allows for the manipulation of the electric charges - in time and space, as done by our invention, as described in the sequel. *The reader is requested to keep this in mind, that the electric pulse propagation within the heart muscle is extremely slow as far as electric phenomena go.*

Several malfunctions are possible to occur that hinder the proper functioning of the heart. Some are of a mechanical nature, a subject not bearing on our invention, while some are of an electrical nature, which is the focus of our invention, as described later on: our invention is an inventive method and means to cause a better propagation of the electric pulse that causes the heart to contract - and consequently, our invention is an inventive method and system to cause a better heart pumping. Better is here used in the sense of pumping more blood for a fixed amount of energy spent for the activity.

There are a wealth of books on the subject of heart contraction. A simple book is Thaler (2003), where the reader with a non-medical background can get more detailed information. In short, if an electric charge is introduced at some point in the muscle, this charge causes a propagating chain of motion of charges, similar to a falling domino sequence, which its associated propagating contraction sequence. This is the mechanism behind the blinking of our eyes, behind our walking, behind the sideways shaking head and the smile of pity of a physician reading this simplistic physicist's view of body cells - and also behind the heart contraction. The heart contraction is an electric driven phenomenon, caused by the injection of the appropriate electrical pulse in the heart muscle at the top of the right atrium and the heart pacemaker is simply an electrode capable of injecting an electric charge at some desired positions in the heart muscle. This will be described in the sequel, and our invention bears on a twist on the man-made mechanism (artificial heart pacemaker) designed to cause an optimized heart pumping contraction sequence. *Our invention improves on the propagation of the artificial electric pulse that causes a heart contraction* (and consequent blood pumping).

As a last preparatory information we want to clarify that the heart pumping mechanism is a modification of a class of pumps called peristaltic pump, which causes the motion of the fluid, or pumping, with a progressive forward squeezing of the container, which forces the fluid forward. If the reader is unfamiliar with the mechanism of peristaltic pumping, we recommend that she/he acquaints her/himself with the method, perhaps observing the animation in the wikipedia article on peristaltic pump, or any similar source. In the experience of one of us (SLPM), the American cardiologists are not generally aware of the progressive forward squeezing of the heart, and that when they state that the heart contracts sequentially they only mean that the atria (top part of the heart) contracts before the ventricle (bottom part of the heart), as opposed to the sequential forwarding squeezing of each cavity. This is partly because the heart moves up and down and also sideways while twisting widely through each cycle, which hinders the observation, but above all because the progressive squeezing is imperfectly made. So, repeating, each heart contraction is a sequential event, in the sense that the muscles start contracting at the extremity further away from the exit port, then sequentially contracting forward, toward the exit valve - as a thoughtful person squeezes the toothpaste tube. This latter sequential contraction is the one the inventors want to bring forth.

One example of a peristaltic pump is the caw milking. Unfortunately very few people have ever milked a caw, including the inventors, so this is a *gedanken* experiment (one of us checked for its accuracy with a farmer, having obtained confirmation of its accuracy). The milker holds the caw's tit between her/his four fingers with the thumb up, near the caw's udder, then progressively squeezes the caw's tit between its pointing finger and the palm of her/his hand, then press the middle finger while holding the pointing finger closed, to prevent back motion of the milk, squeezing the stored liquid further down from the tit, then the annular finger than finally the little finger, all along keeping the previously closed fingers closed to prevent backward motion of the milk. Having pressed the small finger, all the can be squeezed is out, the hand is opened to allow more milk to enter the tit and the process is repeated.

Another example, this one only partly representative of a peristaltic pump, is the toothpaste tube. This example is easier to understand because all readers of this document brush their teeth regularly - or so we hope, so this is more than a *gedanken* experiment! For the best effect, the toothpaste tube should be squeezed from the back on forward. Accordingly, a thoughtful person squeezes the tube from the back end advancing forward as the tube empties, perhaps even rolling the back on itself if the tube is stiff enough to be compatible with this, to close the back volume, forestalling backwards motion of the toothpaste. Scattered minded people squeeze the paste tube from the middle, a practice that drives thoughtful people crazy and have, the inventors suspect, caused many divorces. Squeezing the toothpaste tube from the middle causes a most inefficient toothpaste ejection (pumping). Our hearts, as designed by the American intelligent designer, squeezes the blood out like a scattered-minded persons squeezes the toothpaste tube from the middle, which is the problem that our invention tackles.

The original artificial heart pacemakers simply injected an electric pulse near the sino-atrial node (SAN) at the top of the right atrium, and later versions injected two or even three separate pulses in two or three different parts of the hearts, with the appropriate time delays, which correspond to the elapsed time for the natural pulse to be at that place for a good contraction sequence. None of them, though, even attempted to control the path and the speed of the injected current once it is injected artificially - *which is the object of our invention.* In other words, *our invention improves on the electrical propagation features of the electric pulse created by the artificial heart pacemakers, and in doing so it improves the squeezing sequence of the heart, which in turn improves the pumping efficiency.* It is to be remembered that because the heart is a variation of a peristaltic pump, the pumping sequence is of fundamental importance for an efficient pumping (the inventors hope that the reader did indeed go see the animation in Wikipedia or elsewhere).

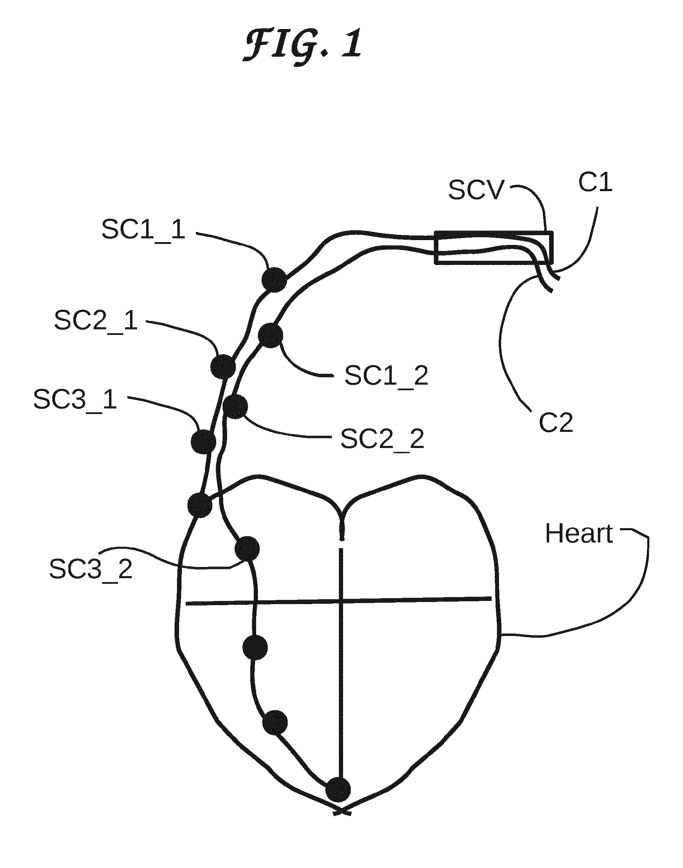

Originally, heart pacemakers were simply an exposed wire wire tip, the wire connected to a battery and electronics circuitry to create pulses of appropriate frequency, duty cycle and amplitude. The original implant was made with an open chest surgery, but this was quickly supplanted by a less invasive and much less traumatic technique, with which an incision was made on some vein at the chest (usually the subclavian vein, **SCV,** FIG. 1, on the upper chest), where a wire was inserted, not much differently than giving blood from the arm's vein. The inserted wire was fitted with an electrode and some sort of anchoring ending at its distal extremity, then this wire was fed into the blood vessel until its distal extremity reached the upper right heart chamber, from the inside (the right atrium), where the wire tip was anchored on the inner part of the heart, near the natural starting point of the electrical pulse that causes the heart to beat, know as the sino-atrial node (SA node or **SAN**). During this process the patient lays in an X-ray imaging system and the surgeon can observe the advancement of the wire down the vein on an X-ray monitor. The proximal end of the wire is then connected to a battery **BAT1** and electronics box **110** which was implanted in the chest, in some convenient location. An electric current emanated from the wire tip anchored at the distal end, which then propagated through the heart muscle, causing the muscle to contract as the current proceeded along it, hopefully similarly to the naturally occurring electric pulse. It is crucial here to remember that this muscle contraction occurs because of the forward propagation of the injected electric charge, and consequently, it is the electric current propagation time and pathway that determines the heart contraction sequence, in time and space - because the muscle cells contract as a consequence of the electric charge arriving to its location. *The sequence of muscle contraction is crucial for an efficient heat functioning,* because the heart must start squeezing from its furthest end, away from the discharge exit area, most away from the exit port, continuously squeezing its walls towards the exit port. The heart does *not* contracts as a person squeezes a tennis ball for exercise, but rather, the heart squeezes sequentially pushing the blood forward, towards the exit port. The reader can here recall the caw milking and the toothpaste tube described above.

Most people get astonished when they learn that the heart of an athlete at her/his peak, pumps only 75% of the blood volume inside it (I was!), that a health young adult pumps only 70% of the blood inside it, then goes down from there until when the heart of an older, inactive person starts pumping less than 50% it becomes time for some intervention. The heart operates with a rather low efficiency! So much for the American intelligent design. Intelligent it was not.

The heart is asymmetric, particularly from the point of view of the point where the stimulating electrode is anchored in the heart, which often is near the sino-atrial node **SAN,** or at the top of the right atrium. It follows that the current that is injected by existing heart pacemakers can hardly be expected to follow well the contour of the heart muscle, much less with a correct timing for a proper squeezing sequence, causing a less than ideal contracting sequence. Other anchoring positions for the electrode are also used, and multiple electrodes as well, which may stimulate the atrium and the ventricle independently and with the appropriate time delays. The multiple electrodes heart pacemakers, also known as Cardiac Resynchronization Therapy, is a step in the right direction of controlling the timing of the start of the upper contraction (the atria) and the start of the lower contraction (the ventricles), but still way too timid, an insufficient step to the need to control the electric charge wave continuously, as it travels through the heart muscle. Cardiac Resynchronization Therapy controls the starting time of the contractions but not the progression in time of them. *What is needed is a device which is capable of continuously controlling the path and speed of the current as it travels through the heart muscle.*

At this point we ask the medical people, particularly the cardiologists and the electrophysiologists to ponder on the need to control the contraction sequence further than only its initial timing, as done by Cardiac Resynchronization Therapy, controlling the progression of the contraction too, all along the heart wall. Electrophysiologists ought to be emotionally prepared to accept this new possibility of controlling the path and progression in time of the injected current.

There exist one exception to the adjustment of the path for the propagating current in the heart, one that shows that the cardiologists are aware of the problem but have not solved it yet - not for the heart pacemakers. This exception of adjustment is the surgery known as catheter ablation, which consists of selectively destroying selected groups of heart cells with the objective of redirecting the path for the propagating currents. Current version of wikipedia (on 21 September 2014) states that: "Catheter ablation is an invasive procedure used to remove or terminate a faulty electrical pathway from sections of the hearts of those who are prone to developing cardiac arrhythmias such as atrial fibrillation, atrial flutter, supraventricular tachycardias (SVT) and Wolff-Parkinson-White syndrome." It is worth to bring this to the attention of the readers because it shows that the problem we solve with our invention is an old known problem which have never been solved, even if so many competent and creative persons have tried to solve it.

Such multiple electrodes, usually worked better than a single electrode. Of course!, after all, they are a step in the direction of controlling the charge motion along the cycle, as opposed to just injecting an electric charge one time only at the beginning of the heart beating cycle. This uncontrolled was shared by most, if not all models of heart pacemaker electrodes in use today, in spite of the fact that the cardiologists are well aware that uncontrolled electric pulse propagation caused inefficient heart pumping. Cardiologists knew that they had to address the problem of electric pulse propagation through the heart, but they have so far not succeeded in this goal. It has been a known problem in heart pacemakers, yet and amazingly, a problem which has defied solution for decades.

It seems that all existing devices, used now or in the past, attempts to solve the problem of electric pulse propagation inside the heart muscle tissues with the use of multiple electrodes, to re-start the propagation mid-way, while nobody succeeded to control the current propagation, in direction and magnitude, which is the object of our invention. Nor have existing devices made full use of multiple electrodes to more completely shape the electric field within the heart muscle - which is the same as the electrical current path, because the electric field lines are the same as the lines of force on the electric charges, or the lines that direct the motion of the electric charges, similar to the steering wheel on an automobile.

Our invention offers a method and a means to adjust the electric field, independently from the stimulating electrodes, to the best shape depending on the particular case, as needed, including controlling the motion after the initial short time when the stimulating electrodes are energized.

OBJECTS AND ADVANTAGES

Accordingly, several objects and advantages of our invention are one or more of the following. A larger electric field produced by the passive electrodes **140_t2** for a given value of the available electric potential (voltage) V, making use of supercapacitors for passive electrodes.

A better squeezing sequence of the heart muscle, starting the muscle contraction further away from the exit port, then progressing to the exit port, with view to achieve a more efficient pumping, when compared with existing artificial heart pacemakers which were designed with no view to optimize the squeezing sequence.

Another object and advantage of our invention is to offer the ability to control the path of the electric current in the heart so as to cause a higher pumping fraction, or the fraction of the blood which is actually pumped out of it, or out of each chamber, when compared with existing artificial pacemakers in use today.

Another object and advantage of our invention is to adjust the electric field over the heart muscle to take better advantage of the atrial ventricular node to cause a better squeezing sequence of the heart muscle when compared with artificial pacemakers in current use.

Another object and advantage of our invention is to adjust the electric field over the part of the heart muscle where the His bundle and the right and left bundles and the Purkinje fibers are, to control the propagation times of the electric current coming from the atrial-ventricular node to the bottom and sides of the ventricle, to cause a better squeezing sequence of the ventricles heart muscle when compared with artificial heart pacemakers in current use.

Another object and advantage of our invention are a better volumetric fit of the neural electrical stimulation to the optimal heart and/or other tissues target volume, when compared with currently used electrical stimulation devices.

Another object and advantage of our invention for brain neural stimulation is to better control the electric field around the supporting structure from where electrical stimulation is injected in the target volume of the brain when performing Deep Brain Stimulation, to cause that the electrical stimulation reaches a larger volume of the target volume while better avoiding stimulating other parts of the brain that are near but outside and beyond the target volume.

Another object and advantage of our invention is the possibility of time control of the motion of charges for stimulation sequences in neural stimulation, which is not achieved with currently used devices.

Another object and advantage of our invention is a better control of the shape of the volume which contains the neurons that receive electrical stimulation in brain stimulation, as in DBS (Deep Brain Stimulation).

Another object and advantage of our invention is a better control of the shape of the volume of neurons that receive electrical stimulation in neural stimulation, as for TENS (Tanscutaneous Electrical Neural Stimulation) pain control.

Another object and advantage of our invention is a better control of the shape of the superficial distribution of neurons as for pain control in TENS (Transcutaneous Electrical Neural Stimulation) devices.

Another object and advantage of our invention is a better control and shape of the mostly planar electrical stimulation of neurons as used in some cortical brain stimulation.

Another object and advantage of our invention is the possibility of better control the volume where the vagal nerve is, to stimulate the vagal nerve and only the vagal nerve, to control blood pressure.

If one or more of the cited objectives is not achieved in a particular case, any one of the remaining objectives should be considered enough for the patent disclosure to stand, as these objectives and advantages are independent of each other.

Further objects and advantages of my invention will become apparent from a consideration of the drawings, the summary, the description of the invention and its variations, and the claims.

SUMMARY

It is well known in cardiology that the heart pumping efficiency is a direct consequence of a proper propagation, in time and space, through all available electrical paths in the heart cells, of the electrical pulse that causes the heart contraction, including the contraction sequence. Included in these cells are the cells of the miocardium, the cells of the His bundle, of the right and left bundle, of the Purkinjie fibers and others. This is acknowledged to be true whether the electrical pulse is the natural one starting at the **SAN** (sino-atrial node) or an artificial one, starting at the anchoring position of an electrode from artificial heart pacemaker - whether it is a single electrode or multiple electrodes. The artificial heart pacemaker does not inject the electric current at the same location as the natural pacemakers, and consequently a well designed artificial heart pacemaker needs to correct for this variation - while the current devices do not correct. Finally, due to the asymmetry of the heart muscle, it would not be expectable that the currently used symmetric electrode would best substitute the natural pacemaker. Consequently, what is needed is a heart pacemaker that could maximize the pumping efficiency. Such a goal has eluded the practitioners because of a lack of mechanism for precise control of the path of the injected electric charge, in position, direction and relative timing. Our invention is a step in the direction of better control of the path and timing of this stimulating pulse. Our invention discloses a mechanism to control the *magnitude and the direction* of the initial current injection in the heart muscle, in space and time, and also time delays between current injected from different locations on the surface of the stimulator *even after the current is injected* in the heart or other organs; in other words, our invention affords the possibility of controlling what we call the "vector current", and the relative time at different directions and places, as opposed to only its magnitude, as in prior art. Our invention also applies to other electrical stimulations as brain (DBS and cortical stimulation), neurons, spine, skin, cochlea and others.

DRAWINGS

FIG. 1. Two electrodes for Cardiac Resynchronization Therapy with multiple supercapacitors at several positions along the cables.

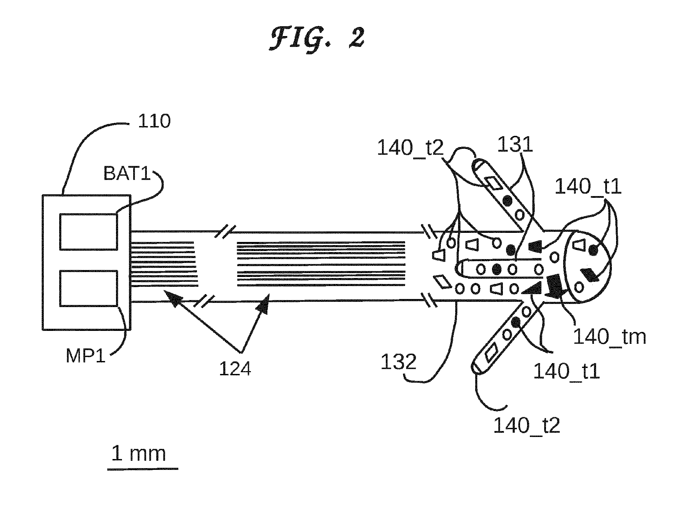

FIG. 2. A heart-type electric pacemaker (piquita) with multiple shaped electrodes connected to the associated battery and electronics. The larger number of connecting wires **124** is for the embodiment with a dedicated wire to each electrode. Other embodiments may have multiple electrodes connected to the same wire together with a selecting mechanism to select the electrodes that are connected to the battery / controlling electronics.

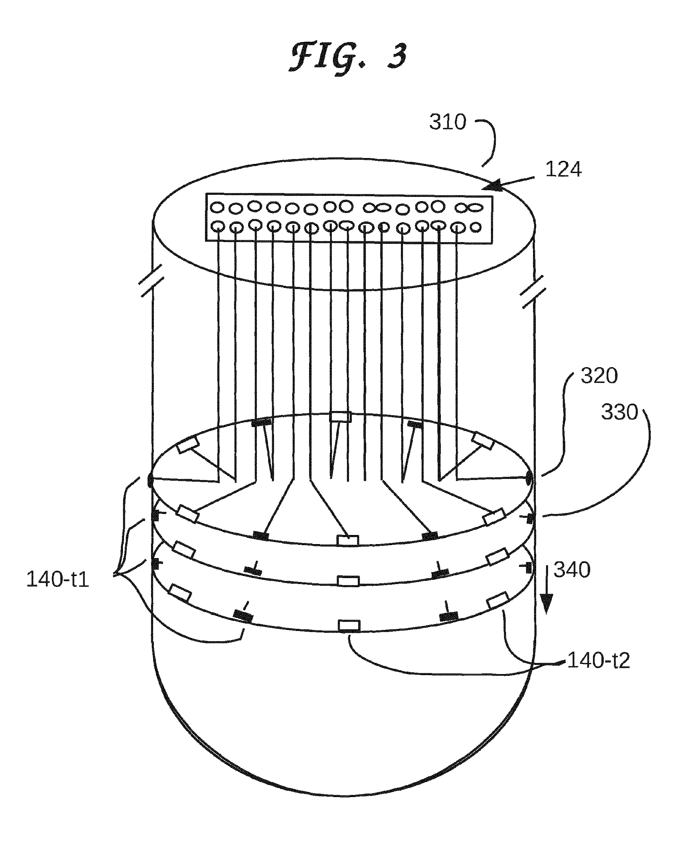

FIG. 3. Shows a perspective view of a picafina brain-type stimulator of our invention showing a schematic view of the inner wires and connections. Not all wires in the vertical direction are shown, for simplicity, but only the wires that connect to the top layer of electrodes plus a few more to lower layers. This embodiment uses one dedicated wire for each electrode and both type-1 **140_t1** and type-2 **140_t2** electrodes.

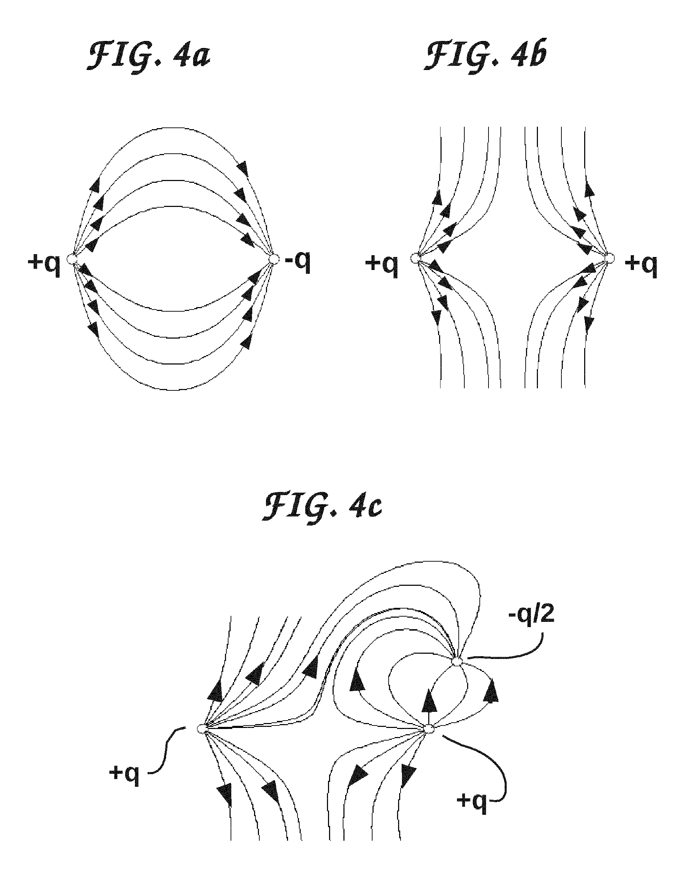

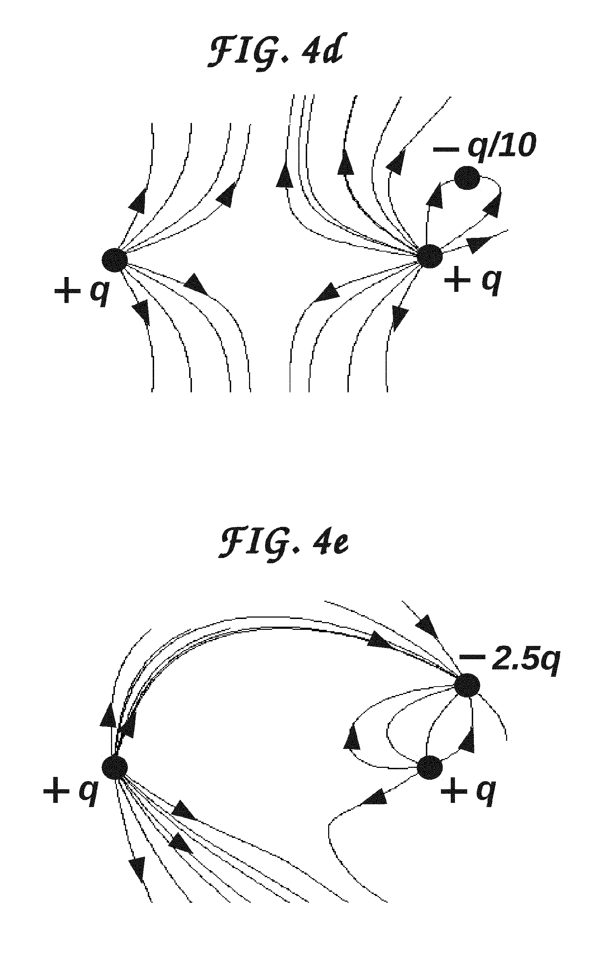

FIG. 4(a, b and c). Three examples of electric field lines (which are the lines along which a positive charge would move). The field lines differ for different electric charges due to their sign (+ or -), numerical value (q, q/2, etc.) and position in space. The reader should notice that such slightly differences in charges produce vastly different shapes of the electric fields, which are the paths of charges free to move in the space in each configuration. The space may be around a heart, for example, so each charge distribution causes a different heart contraction sequence, because the electric charges would move following a different path (causing different muscles to contract) and at different speeds, causing a time delay characteristic of each speed - as much as different cars moving towards different directions and at different speeds would arrive at different places and at different times..

FIG. 4 (d and e). Effect of changing the numerical value of the electric charges, which is equivalent to modifying the electric potential (or voltage), with the same spatial configuration of two positive and one negative charges at the same locations. The reader will notice how vastly different the field lines are with a simple change of one charge from a small value of q/10 to a larger value of 2.5 q.

FIG. 5. Shows a schematic representation of a brain-type picafina of our invention, with some of the electronics that controls its functioning. Similar designs apply to the heart-type piquita and other variations.

FIG. 6. Resistor network similar to current path in cells but with denumerable paths.

FIG. 7. Shows a schematic connection between the sealed box 110 containing the energy storage unit BAT1 (battery, etc.), the microprocessor MP1, the necessary electronics, the electrical connecting means and a brain-type picafina stimulator of our invention. Similar connections are valid for the heart-type piquita and other variations.

FIG. 8. The gravitational field of the planet Earth showing an exaggerated sideways deformation due to mountain **m.**

FIG. 9 (a and b) two supercapacitor rings to focus (a) and defocus (b) a charged particle beam in a charged particle accelerator.

DETAILED DESCRIPTION

In the following we are introducing some terms with precisely defined meaning which we clarify here: these are passive electrodes, active electrodes, supercapacitors, picafina, piquita, planarium and tine. **Passive electrodes** are electrodes which are capable of creating electric field lines, but *not capable to inject electric current* in the space surrounding them. Physically, and this is most important for this patent, what characterizes an electrode as passive is that it is covered by an electric insulating layer. Passive electrodes are disclosed in the European patent application EP 2012 0167688.6 and in issued US patent US 8,954,145. *This patent application is for the use of supercapacitors for the passive electrodes, with the objective of enhancing the electric field created by the passive electrodes.* The insulating layer prevents any electric charge from leaving the electrode to the outside of the supporting structure, whether a picafina, a piquita, a planarium or any other type. The reader should keep in mind that electrical insulators do not prevent the electric field lines from existing past the insulating layer covering the passive electrodes, but only prevent the electric charges from penetrating them, and, consequently, the passive electrodes are perfectly capable of creating field lines in their surrounding volume, e.g., in the heart muscle. Aside from this, passive electrodes work best for their purposes the larger the electric charges they can hold, because the electric field is determined by the value, or magnitude of the electric charge. Consequently the passive electrodes should be capable to store the maximum amount of electric charge. They are preferentially larger capacitors, generally of the class known as supercapacitors, because what is meant by "capacitance" is the capacity to hold electric charge. In this account the passive electrodes are largely different than the electrodes in use today, because the passive electrodes are made to maximize the charge stored, as it will become clear as their function is further discussed. They are labeled as **140_t2.**

**Active electrodes** are the ordinary electrodes **140_t1** of the electrical stimulators used by current devices: they are capable of injecting electric charges in the space surrounding them, and these charges are then capable of moving in the medium surrounding the electrodes. They are labeled here as **140_t1.**

**Picafina** is a elongated penetrating supporting structure of cylindrical shape, of the type generally used for Deep Brain Stimulation, measuring a few centimeters in length by one-plus millimeters in diameter (for example, 7 cm in length by 1.3 mm in diameter), which encompass other forms of stimulators too. Picafina is what is usually called by the companies by the general term "lead", which we avoid for being misleading. The picafina of our invention typically has several active stimulating electrodes at its extremity and passive electrodes distributed over its extremity and over its whole body as well. Cf. with piquita and planarium.

**Piquita** is a penetrating supporting structure which often resembles an anchor (as a shipping anchor), with a small penetrating structure with some few anchoring tips, called "tines", which serve to prevent the piquita from dislodging from the tissue where it has been inserted. Other shapes are possible too, and we use the term to indicate electric stimulators designed for use in the heart. Piquita is designed to be used for stimulation of the heart, where it is implanted on its inner side where it is inserted from a vein, typically the sub-clavian vein, with no harm to the person who receives it. It is currently also referred to as a "lead", in spite of its form being so different than the brain electrode supporting structure. Cf. with picafina and planarium.

**Planarium** is a structure which is designed to support a number of stimulating electrodes on a planar supporting structure which may be attached to the skin of a person or to the outer surface of an organ (as to the pericardium of a heart or the outer surface of a brain). Cf. with picafina and piquita.

**Supercapacitor** is an ill-defined term used in the electronics world to indicate an ordinary capacitor typically made with late 20th-Century technology, typically, but not exclusively involving a large number of small cavities in the bulk of the material, with the consequence of a very large increase of the surface area of the material, and typically meaning a capacitor with capacitance measured in Farads, which was beyond the wildest dream a few decades ago. We cannot define the term better than the electronics usage of it, but for us the term means what a typical electronic engineer would consider a capacitor with a truly large capability of storing electric charge, typically, but not restrictively so, in the value of several Farads. The larger the electric charge capability the best. When necessary, they are labeled as **SC1_1, SC2_1**, etc. , but often they are just referred by their functions as passive electrodes and then referred as **140_t2.** Preferably passive electrodes are more than a metallic surface covered by an insulator; under the insulating layer it is preferable to have a supercapacitor than just a metallic surface.

**Tines** are anchoring arms **131,** generally at the tip of the piquita (heart electric stimulator), similar to the grabing arms at a ship's anchor, which serve the same purpose as the ship's anchor: to prevent the piquita from dislodging from its insertion place in the heart. Many piquita in use today have four tines but this number is not required.

FIG. 2 shows the main embodiment of our invention, which is for heart pacemaking applications, which we call piquita. FIG. 2 shows one of the current art anchoring distal extremities **132tip** of a current used heart pacemaker, with the improvements of our invention. Note that different ending anchoring attachments **131** are in use, and that the model shown in FIG. 2 uses one of the several used attachment endings, but the same principles apply to other anchoring attachments. The main body **132** of the piquita device may have a diameter of 3 mm or less, as 2 mm or 1mm (approximate dimensions), and the smaller anchoring side arms **131** may have a diameter of 1 mm or 0.5 mm (approximate dimensions, the actual dimension being unimportant to the invention). Anchoring arms **131** should have such size and strength enough to keep the tip of the stimulating piquita structure **132** secured in place once it is inserted into the heart muscle from the inside of the heart. These dimensions may vary without changing the nature of our invention and these values are given as a possible dimensions only. On the surface of the main body **132** and of the smaller side arms **131** there are several random-shaped patches which are represented by either a solid black or a white shape represented by its contour. The solid black odd-shaped patches **140-t1** represent electrodes which we call active, or type-1 electrodes, and the open, odd-shaped patches **140-t2** represent electrodes which we call passive, or type-II or type-2 electrodes. These type-2 electrodes is one of the main inventive characteristic or our invention. They are also described in our patent number US 8,954,145, which discloses a more complex embodiment of the invention disclosed here. The invention disclosed here does *not* use the local addresses near the electrodes, having instead a large number of wires connecting the electrodes to the battery **BAT1** / controlling electronics-microprocessor **MP1,** one dedicated wire for each electrode. The invention disclosed here has one less element than the invention disclosed in 8,954,145, but this invention works either way, the particular method of making the electrical connection not being part of the invention.

FIG. 3 shows a perspective view of the brain-style (a.k.a. Picafina), with some wires down the length of the device, but not all wires to prevent cluttering the drawing. Only the wires that make the connection to the electrodes at the top layer **320** are shown. Other electrodes, on the layers below (**330, 340,** etc.), are also connected to dedicated wires, similar to the ones shown in this figure. In the main embodiment the wires are of the printed circuit type, but lose wires are also possible, though a smaller number of them would be possible. At the top of the brain-type picafina shown in FIG. 3 there is an electrical connector, which is capable of matching another connector (as male-female type) with wires leading to the battery **BAT1** and controlling electronics/microprocessor **MP1** implanted at another location, inside sealed box **110,** as in devices in use today.

Active, or type-1 electrodes **140-t1** have a metallic surface which is capable of conducting electricity. Other than their smaller sizes and odd-shapes, they correspond to the electrodes in use today (prior-art electrodes in patent jargon) for electrical stimulation of the heart, brain, and other body parts. It is worth to mention that though the size and configuration of the electrodes disclosed here add to their functionality, part of the improvement disclosed here is also achievable with larger electrodes as used by current electrical stimulators. Passive, or type-2 electrodes **140-t2** besides being preferably made with supercapacitors, their surface is covered by an insulating layer, which, in the main embodiment is made of silicon oxide but other insulators are acceptable for the functioning of the invention. It is worth to mention that passive electrodes may be simple metallic surfaces covered by an insulating layer, totally similar to active electrodes, just that they do not function as well. Passive, type-2 electrodes are unable to inject current into the surrounding tissues, but when set at fixed electric potentials (voltages) they do change the shape of the electric field in the neighborhood of the piquita, therefore changing the paths of the injected currents. Passive (type-2) electrodes are incorporated in the piquita for the purpose of field shaping (to change the spatial configuration of the surrounding electric field which in turn changes the path of the electrical stimulation). Examples of field-shaping are shown in FIG. 4 (a, b, c, d and e), which display several different electric field configurations for different electric charge distributions.

As stated above, the electric field is a function of the electric charges at different places, not of the voltages at the places or a function of the current emanating from the places, so the passive electrodes are preferably, but not necessarily constructed with the technology of supercapacitors (see definition above) to maximize their impact on the electric field created by them. This is so because the electric field is governed by Coulomb's law:

$$\mathbf{E} \text{(vector)} = k * ( Q / r\text{^}2) (\mathbf{r}\text{-hat}), \hspace{3cm} \text{(EQ\_Efield)}$$

Where **E** is a vector (most patent publications are not set to the conventional boldface, so we add the word "vector"), k is a constant of proportionality described in most elementary books on electricity and magnetism, Q is the electric charge which creates the electric field, r is the distance (scalar, just the number) from the charge **Q** to the point where the field is calculated, and **r**-hat is a unit vector, which gives the direction to the field **E** on the left-hand-side, without affecting its magnitude, known as unit vector. In mathematical texts **r**-hat is indicated by a boldface **r** with a hat on top of it to indicate it is a vector of unit length. There are conventionally accepted norms governing the direction of **E** and other peculiarities of the vector **E** which we are swiping under the rug for conciseness.

Observing the Coulomb's law above it is seen that the electric field **E** is directly proportional to the electric charge Q, so the larger the charge Q the larger the electric field **E** is. Since the force **F** on the charge injected by the active electrodes is proportional to the electric field **E,** it follows that a larger electric field **E** causes a larger force **F** on the injected particle and can, therefore, have larger influence on its motion (as a larger engine car offers more options to the driver when compared with a smaller engine car).

Now, the value of the charge that a particular battery can "pack" into the electrode depends on the "force" or "strength" of the battery, which is measured by its electric potential (unfortunately called voltage in US) and a few geometric and space characteristics which are lumped in a quantity called capacitance, usually indicated by the letter C (capital C):

$$Q = C * V$$

It follows that one can arbitrarily increase the charge **Q** that creates the electric field **E** (and consequently the force **F** on the electric charge injected by the active electrode) by arbitrarily increasing V or increasing C. Unfortunately V is created by a battery, so it cannot exceed the electric potential of the battery, which normally is a few volts only, as known by most of us (just think that most of the batteries we ever handle are 1.2 V or 1.5 V, the 12V car battery being actually six 2V individual batteries (called cells in this case) on "top" of each other to add to 12V). Consequently, given that V in the equation above is limited to a rather low value, it is left to see if C can be increased. It turns out that until recently C was also rather restricted in maximum value, but recently a new class of capacitors, baptized as "supercapacitors" do sport an enormously large value of C. In the case of the supercapacitors their capacitance value (C) is increased using modern technology that largely increases the surface area of the device, a porous surface under the insulating layer.

The part of the main embodiment of our invention which preferentially (but not exclusively) uses a supercapacitor for passive electrodes does so to boost the numerical value of the stored charge "Q", which in turn increases the value of the magnitude of the electric field **E,** which in turn increases the magnitude of the force **F** imparted on the electric charge injected by the active electrode. Of course that not necessarily a very large value of **F** is required, particularly all over the place. What our invention offers is the possibility of a large force **F** when one such is needed. Moreover, for the same required force **F** a much smaller electric potential (voltage) V is required if the passive electrode is characterized by a large capacitance C, therefore decreasing the requirements on the valuable battery that nobody wants to replace (with a surgery!).

Another part of the main embodiment, with the same objective of increasing control on the electric field **E** is the introduction of the type_2 passive electrodes **140_t2** with a large plane shape which we call planarium. These electrodes, possibly using the supercapacitor technology, but not necessarily so, are manufactured in the necessary shape to be implanted in different parts of the patient's body. The planaria differ in shape from the picafina and piquita in that planaria are generally flat, perhaps with a curvature to conform to a shape but still sheet-like in general shape. For example, a planarium could be manufactured to cover the outer contour of the heart, just outside the pericardium, which is the sac that contains the heart. We call it the pericardium planarium. Such a planarium would have a strong control on the electric field inside its volume, but it would require an open-heart surgery to implant, which is highly undesirable. It may become more acceptable in cases where, for some reason, the patient is already undergoing open heart surgery anyway, in which case the pericardium planarium could become a viable choice. Other designs are less invasive than the pericardium planarium, but also less able to control the electric field in the heart muscle.

Another option is to implant planaria just below the skin, at the chest, side of thorax and back of the patient. We call these under skin planaria. These would require less invasive surgery, at the cost of being less effective for being more distant from the heart than the pericardium planarium. One or two under skin planaria could be implanted near the implantation of the sealed box **110** at the initial implant time, using the opportunity that the patient is already opened up anyway. Such a planarium would offer a smaller surface area but it would be implanted at no extra surgical effort, so it could become a viable choice.

Another option, which is still less effective than the under skin planaria is to make passive electrodes attached to a tight shirt-like cover for the thorax, as a tight T-shirt, which is connected to a battery conveniently located, say, at the bottom of it, connected by wires to the electrodes at the inner surface of the shirt-like wearable device. We call this the shirt-like planarium. The shirt-like planarium offers still less effective control of the electric field when compared with all the implanted types, but they offer the advantage of requiring no surgery at all, including for battery changes. This shirt-like planaria could be just at the front of the chest, or just at the sides of the torax, or just at the back, or any combination of these. The positive side of the shirt-like planaria is that they could surround most of the volume around the heart, almost half of the spherical solid angle with the heart at its center, which is a good advantage.

Still another variation of shape for the type_2 electrodes **140_t2** is to manufacture type_2 electrodes on the length of a wire which is designed to be inserted into the chest using laparoscopy (that is, minimally invasive surgery through a small hole in the body). The effectiveness of such a line of type_2 electrodes would be even less than a under-skin planarium because it would offer less surface area, but it would offer the advantage of closeness to the heart. If effective surgical techniques were developed to perform such a surgery it may become a good choice due to laparoscopy being minimally invasive surgery. In principle techniques could be also devised to insert a planarium via laparoscopy too, probably not to cover the whole heart as the pericardium-type planarium, but still stretching near the heart on some of its sides or in front or back of it.

As the reader will see, several types of planaria-type electrodes can be devised to control the electric field in the heart muscle with different effectiveness and different levels of difficulty and surgical danger that have to be weighted by the surgeon and by the patient on a case-by-case basis.

The invention also discloses a marker to determine the angular position of the piquita with respect to the heart (or brain, or nerve, etc.) in which it is implanted. FIG. 2 shows one such possible marker: a type-1 active electrode **140-tm** with such an X-ray opacity (absorption or scattering cross-section) to be visible during the fluoroscopic images taken during electrode implantation as normally done. Other markers are possible for the same purpose, as the same shapes on type-2 passive electrodes, as side arms **131** of different lengths and/or diameters, or any other asymmetric feature that is visible in some sort of imaging technique, as MRI, X-ray, ultrasound, etc. It is part of our invention that each electrode position and size and orientation is known to the cardiologist (and the computer which he will use to program the device), each electrode being know by a number, as 1, 2, 3, ... etc., or any other identifying pattern. Marker **140-tm** allows for the computer program to know the angular position of each electrode, which is needed to determine which individual electrode to connect to which voltage, according to their actual position within the heart muscle, as the piquita happened to have been anchored in it.

Inside the main body **132** and the side arms **131** of the piquita supporting structure, there are wires **124** extending from the controlling electronics, microprocessor and battery to each electrode **140** (of either type, t1 or t2). Wires **124** may be either standard wires or may also be printed wires, as in printed circuit boards, in this case more likely printed on a flexible plastic support but any of the existing technologies are acceptable, this patent being not on the printed circuit technology. The technology of printed circuits is a well advanced technology with many methods to print the wires, and the wire manufacturing is not part of this invention, as any of the existing technologies are acceptable to implement the invention. These wires may carry analog or digital information, both options being compatible with our invention. Our patents numbers US 8,335,551, 8,538,516, and 8,565,868 disclose digital methods and means to integrate the electrodes disclosed in this patent application.

The random placement, shape and size of the electrodes is a distinct feature of our invention, as it contributes for the creation of a spatial asymmetry of the electrodes, which in turn causes an asymmetry in the spatial distribution of the injected current, either its magnitude or its direction or both.

FIG. 1 shows one of the features of the main embodiment of this invention, which is the supercapacitors **SC1_1, SC2_1, SC3_1**, etc along the wires or cables **C1** and **C2.** FIG. 1 shows a heart with its 4 chambers and two wires or cables **C1** and **C2,** on which a number of passive electrodes **140_t2** are located. In this case the passive electrodes **140_t2** are made as supercapacitors **SC,** but variations with simple metallic surfaces for **140_t2** are possible. Generally speaking, the passive electrodes are distributed over as wide a volume as possible, within the constraints of the surgery and the location of the device, to have more control on the electric field, described by equation (EQ_Efield), as will be understood by the electrical engineers and physicists. In the case indicated in FIG. 1 they are distributed over the length of the wire/cable **C1** and **C2.** The wider is the distribution of the passive electrodes **140_t2** over the body of the patient, the stronger is the control that the microcontroller **MC1** has on the value and direction of the electric field that eventually control the direction and speed of the electric charges injected in the heart (or other body part). The passive electrodes **140_t2** (which in the main embodiment are supercapacitors **SC**) preferably should be located near the volume where the electric charges are moving. This is a consequence of the mathematical dependence of the electric field **E** (see equation (EQ_Efield)) on the distance to the place where the field is, which decreases with the square of the distance, so, for passive electrodes located at large distances from the desired location the contribution is smaller than the contribution by another passive electrodes located closer to the desired location.

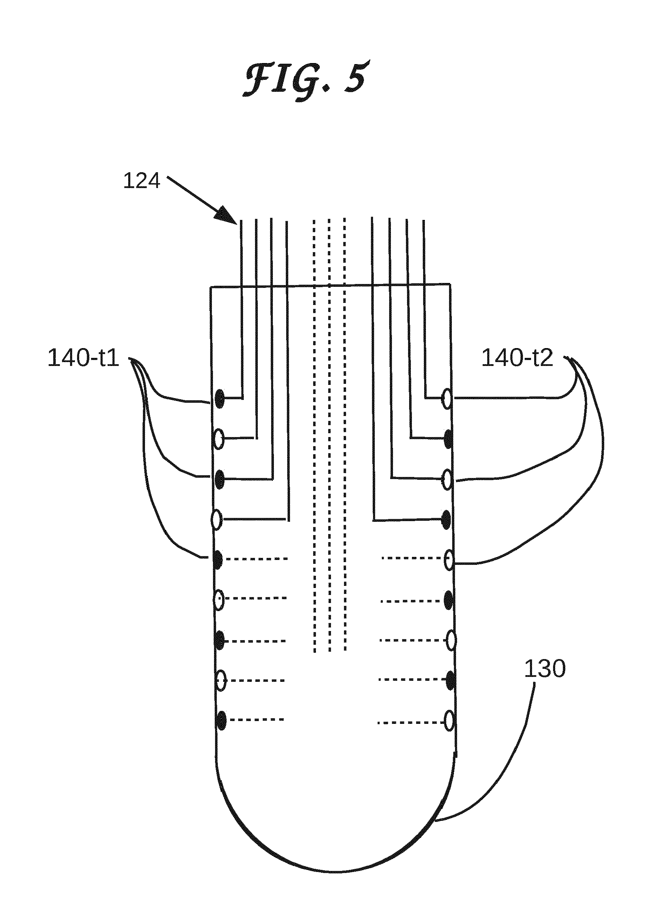

FIG. 5 shows one of the possible ways to connect the electrodes with dedicated wires.

OPERATION OF THE INVENTION

**Background information on operation of the invention.**

Knowledge from two distinct fields are necessary to understand our invention. Firstly it is necessary to understand the mechanism of heart pumping from the cell/muscle point of view, usually an area studied by medical people, cardiologists and electrophysiologists. Secondly, it is necessary to understand the mechanism of propagation of the electrical charge that is associated with the contraction of the cells that make the heart muscle, usually an area of knowledge studied by physicists and engineers. Since this invention involves knowledge from two so different fields of knowledge, namely electrical engineering & physics and medicine & physiology, each part of the description needs to be detailed enough to be understood by someone with little or no knowledge on that part, whether it is an electrical engineering concept, unfamiliar to a medical person, or a cellular physiology concept, unfamiliar to an electrical engineer.

The walls of the heart vary in thickness at different parts of the heart (as much as the biceps is of different volume than the triceps on the arm), and each part varies differently as the person ages (as the biceps changes volume for the same reason). The pumping optimization has also to do with the local resistivity of the heart muscles through which the electric pulse propagates, which changes with time throughout the lifetime of the person. The local resistivity is most important for the correct muscle contracting sequence, because it is responsible for the contracting sequence. *This is where the invention enters: a method and a means to apply a particular electric field as a function of space and time to compensate for the change in local resistivity of the heart muscle and still cause an appropriate contraction sequence.*

The heart being a little taller (that is, along the person's vertical direction) than wider, let us further define a "vertical" axis on the heart as an axis passing through its almost vertical direction, even though the heart is neither quite vertically aligned nor that much "taller" than "wider". We will call this the heart z axis **Z_axis** at its center, with two other axis through the centerline of the left and right sections of the heart, which we call **Z_left** and **Z_right.**

As is known by the medical people, and particularly the electrophysiologists, the heart contracts sequentially in the sense that each chamber initiates a contraction sequence at the extremity that is opposite to the exit port, then progressively contracting cells that are located closer to the exit port, until the cells most near the exit port, when the cycle completes and stops. One such cycle occurs at the upper chamber (the atrium) followed by another such cycle at the lower chamber (the ventricle), then the whole process repeats: atrium-ventricle-atrium-ventricle... For this cycle to occur in an optimal sequence, the electric charges that propagate through the heart muscle must propagate at equal speeds all around the heart, so that they advance together all around the heart, front, back, left and right, around the z_left (or z_right), producing a progressive circular squeeze on the heart, as a chef does on a long, cylindrical, thinskinned package of pate, as he forces the pâté out of the tube. Moreover, each part of the heart hopefully contracts with a strength proportional to the required force for the particular chamber and the level of blood supply needed at the moment, which depends on many physiological factors, as physical activity, emotional stress, and so on. For the ideal heart the current density should proportional to the local required pumping strength - it would be so for an ideal heart, but unfortunately it is *not* so in the real heart. Again for an ideal heart, the wall thickness should be proportional to the required pumping strength - this is approximately true, the ventricules being thicker than the atria, and the left ventricule having thicker walls than the right ventricule. Finally, the ideal heart, should have a contraction equally strong all around the heart, that is, at the same values of the coordinate along z (left and right, the z_left / z_right axis), so that there ought to exist a higher electric current where the heart wall is thicker, because there are more cells to stimulate there, causing the same current density (the same force). Unfortunately most hearts, even very good ones, fail to keep a good electrical pulse progression, resulting in non-optimal heart contraction and consequently in smaller pumping fraction (smaller volumetric fraction of the blood pumped forward). Advancing the conclusion, our invention addresses this problem of muscle strength of contraction all around the heart with the objective of improving the volumetric pumping efficiency.

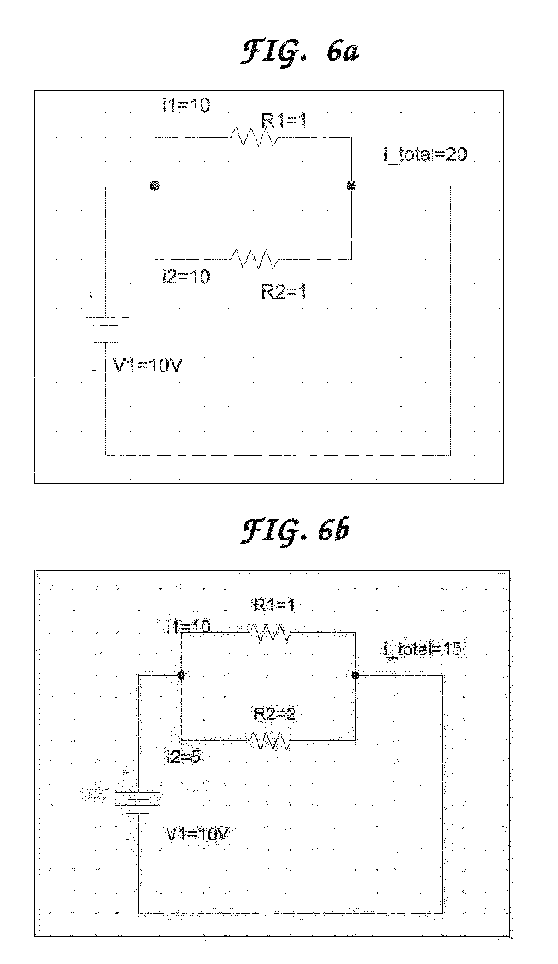

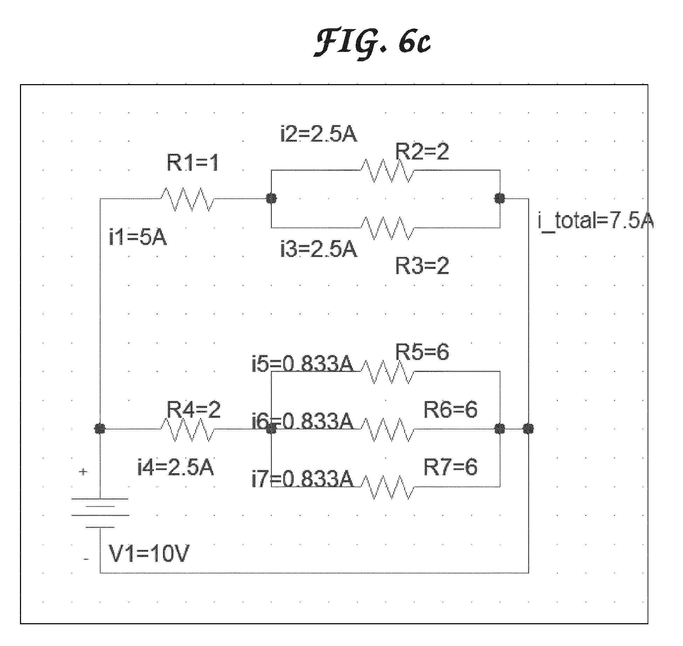

As a simpler example of the splitting of currents through different parts of the heart we invite the reader to go through a simpler current splitting, one that is usually part of the homework of standard high-school in Europe (including Soviet Union), South America, Africa and Asia. The numerical values were chosen to make the calculated values easier to manipulate, even if unrealistic, avoiding micros, millis, kilos and megas, for the benefit of the readers with less technical background. The technically minded reader is asked not to get disgusted by the unrealistic high values of currents and the unrealistic low values of resistances. Figures 6a, 6b and 6c show three resistors networks connected to a battery. FIG. 6a shows two resistors of equal value, R1 and R2, in parallel (1 Ohm each) connected to a battery with e = 10V. Standard electrical network calculations shows that the current in each resistor is 10 A (we omit the calculation here for sake of space and focus). The resistors being equal in value, it is expected that the currents through each is the same, as calculated. This is the situation of currents distributing throughout a perfectly symmetric heart, not a realistic heart. FIG. 6b shows two resistors of different values, R1 = 1 Ohm and R2 = 2 Ohms. In this case electrical network calculations calculate that the current in R1 is i1 = 10 A and that the current in R2 is i2 = 5 A. Common sense cause that one expect that R2 being twice as "hard", or twice as "difficult", should allow a current that is half of the current in R1, as calculations predict. This is the situation of currents distributing throughout a little more realistic heart, the left side of which using more current than the right side of it. Then, finally FIG. 6c shows a little more complex network with currents as shown at table T1, where the subindex of currents match the subindexes of resistors, that is i_1 is the current through R_1, etc. This corresponds to a more realistic approximation in that there are current subdivision on top of current subdivisions - again warning that current values in the heart are much lower, resistance values are much higher and the battery being less than 10 V, the numbers being chosen only to illustrate a concept, not to be realistic. The reader is now asked to extrapolate from this to a continuum situation, in which a battery applies an electrical force (a voltage, so to say) at the top-right of the heart, the sino-atrial node (SAN), which then spreads through the heart muscle, downwards, through not two resistors, as in FIG. 6a and 6b, not through 7 resistors, as in 6c, but through a network of zillions of a continuum of resistors, each offering a different resistance, according to the particular state of health of each cell, including their past history, as scars due to small infarcts and heart-braking events in the youth, fat due to couch-potatoing, genetic defects and so on, so that the current in each cell is different from the neighboring cells. A different current would flow through each path, faster or slower according to the easiness or difficulty of motion, faster here, slower there, all the while the cell contraction occurring at the arrival of the electric charge with a strength proportional to the current i. Most people think about the contracting heart as an extension of FIG. 6a, with equal resistances at all paths, so the currents spread equally and flow at the same speed with an even contractions forward, but in the real heart the resistances are not equal, so the currents spread unequally and flow at different speeds with an uneven contraction sequence. This is the unstated assumption held by everybody that the heart contracts evenly, even though any cardiologist will immediately acknowledge that the cells at the miocardium ought to have very different electrical characteristics, there included their resistivity and their capacity to contract, the force that each can impart to a contraction cycle.

To understand the operation of our invention, the reader must keep in mind what causes the heart to contract, and therefore to pump the blood, and the sequential nature of this contraction, which is a consequence of the progressive motion of the electric charges through the heart walls, mostly the miocardium.

The heart muscle contraction occurs as a consequence of and together with the propagating electric pulse that moves in 3-D (three dimensions) through the heart muscles. As the electric charges propagate through the heart muscle, reaching new cells, each cell suffers a contraction event as the electric charges reaches it, one cell after another, in sequence. The moving electric pulse can be seen as a 3-dimensional extension of a falling domino event, each falling domino piece causing the fall of the piece ahead of it, the whole sequence propagating as a wave. The wave is easy to visualize in a domino falling sequence, because it is 1-dimensional, along the line of domino pieces, so I urge the reader to image such a wave propagating in 3 dimensions through the heart muscle and causing the muscle to progressively contract as the electric pulse propagates. This progressive nature of the electric charge motion and of muscle contraction should be kept in mind. Another analogy, this time in 2 dimensions is a circular wave on the surface of still water, propagating outwards from a point where a disturbance occurred, as a stone dropped on the water. The heart mechanism can be seen as a wave too, but in 3 dimensions. Moreover, the heart contraction is quickly followed by a decontraction event, which is the equivalent of the fallen dominoes raising up after the falling wave passes, which does not occur with the dominoes, or one can think as a person raising the domino pieces again after the wave passes.

Besides the directional electric current flow, which is started again at every heart beat at the sinoatrial node, the local reactance plays a role, as it determines a 3-D continuous network which determines the time delay and magnitude of the local electric pulse, which in turn determines the local timing and strength of the local squeezing. Incorrect time delays of the electric pulse are costly for the pumping efficiency, because they are the very cause of the muscle contraction, that is, of the pumping, and localized higher or lower resistivity are costly too, because they change the electric current intensity, which in turn decrease or increase the strength of the muscle contraction, that is, of the pumping pressure, either way decreasing the total pumping volume. For example, in locations where the resistivity is larger, the smaller current resulting from the higher resistance can be restored to the ideal value by a larger local electric field, because this latter causes a larger force on the moving ions. The reader can appreciate that if the left wall of a ventricle contracts first (while the right wall not), then the heart would simply move to the opposite direction (to the right), as a whole, with no or little internal contraction. If then, later, the right wall of the same ventricle contracts (while the left wall does not, having by then completed its contraction), then the whole heart would move in the opposite direction again (to the left now), as a whole, and again with no or little internal contraction. In fact it is known that such a motion is common, as shown by many videos of an *in vivo* beating heart.

Taken together, controlling the direction and the magnitude of the current, our invention is capable of controlling the position and the magnitude of the squeezing sequence, that is, forcing that the ions propagate around the heart muscle in such a way as to cause a ring of contraction to propagate from the side away from the exit port to the side of the exit port.

This sequential contraction, characteristic of all peristaltic pumps, is similar to the process of squeezing toothpaste out of the tube: it is a *progressive squeezing sequence which progress from the back to the exit port*, as opposed to a simultaneous contraction from all sides as a person squeezing a tennis ball with one hand holding the ball for exercise. Granted that there are people that extract the toothpaste squeezing the tube from the middle, but it is universally acknowledged to be inefficient to do so, even by the very people that do it; they make a huge mess and drive other family members crazy trying to fix it all the time. The inventors suspect that many a marriage ended in divorce because of such improperly squeezed toothpaste tubes. It would be ideal if the heart squeezed as a properly used toothpaste tube, not as a collapsing air balloon that collapses upon itself from all directions at the same time, but alas, the heart is far from a good pump. The heart is not as good as it should be at squeezing from back to exit, the American intelligent designer was not that intelligent after all, and our invention improves the heart contraction sequence, directing it to go into a properly sequential squeezing.

This said, the reader should keep in mind two important points here which is the detail on which the whole invention hinges, and which we urge the reader to pay attention and ponder on. Firstly, that not only is the heart contraction caused by an electric pulse but also that this electrical pulse propagates relatively slowly through its muscles and special fibers, because it relies on the propagation of heavy ions in a viscous medium. The propagation of this electrical pulse is very slow as far as electric events happens, the whole process taking just below one second to complete (at a normal heart beating rate of 70 beats per minute). This means that the times involved are of the order of 10s and even 100s milliseconds for each part of the cycle, the full cycle taking around 900 milliseconds. This slow propagation time is important for our invention to work, as it will become evident in the sequel. The much faster propagation of electric charges in wires and transistors (1 million times faster), allows that a human-engineered circuit can take over the natural process and improve on it - a very interesting project indeed!

As we have shown above, the good contraction sequence of the heart depends on the correct propagation of the electric current, because the latter determines the former. The electric current propagation, in turn, depends on the electrical characteristics of the diverse muscles (cells) which comprise the heart, including rapidly electric propagating cells (His fibers, Purkinje fibers, etc), endocardio and miocardio cells, all of which suffer individual variations from person to person, due to their genetic make-up, to which other variations accumulate during the person's lifetime, due to his exercise and eating habits, etc, to which unlucky events as small localized infarctions in the person's later years and heart breaking events in the person's earlier years, all adding scar tissues with different conductivity and loss of contraction capability, all adding to a conceptually simple problem, yet of complex analytical solution. This, in turn, is the problem which our invention address: how to better adjust the 3-D electric current propagation through the heart in order to cause the best heart squeezing sequence possible for a particular individual, given his possibilities as determined by the physical conditions of her/his heart.

Another way to say the same thing is to notice that unlike a standard electrical network, on which the paths are discrete and fixed, the electrical path for the current that produces the muscle contraction is continuous over the whole 3-D structure of the heart, and some leak out of it too - these are the pulses measured as EKG signals at the chest surface and even arms and legs surfaces. Because the former, a standard electrical network is composed of discrete, enumerable paths, the information is given as the denumerable branches and nodes, while in the latter case (the heart) the information is a continuous current vector field. For the brain the situation is a little simpler but still it is a continuous path, likewise for other organs, as stomach, bladder, etc.

Besides selecting which electrodes are turned on or off (connected or disconnected from the electrical power), the controlling microprocessor **MP1** may, in some incarnations of the device, but not necessarily always, also select different values of voltages to be connected to the electrodes, both active and passive electrodes. Varying the voltage at the passive electrodes changes the magnitude of the charge deposited on the passive electrodes, therefore changing the electric field in its neighborhood, and therefore adjusting the force on and consequently the path of the electric current that was previously injected by the active electrodes. This offers an advantage over currently used heart pacemakers because our invention can better direct the electric current to the particular desirable target volume and avoid entering into undesirable volumes. Also, varying the voltage at the active electrodes, the device can adjust the magnitude of the current that is injected into the heart.

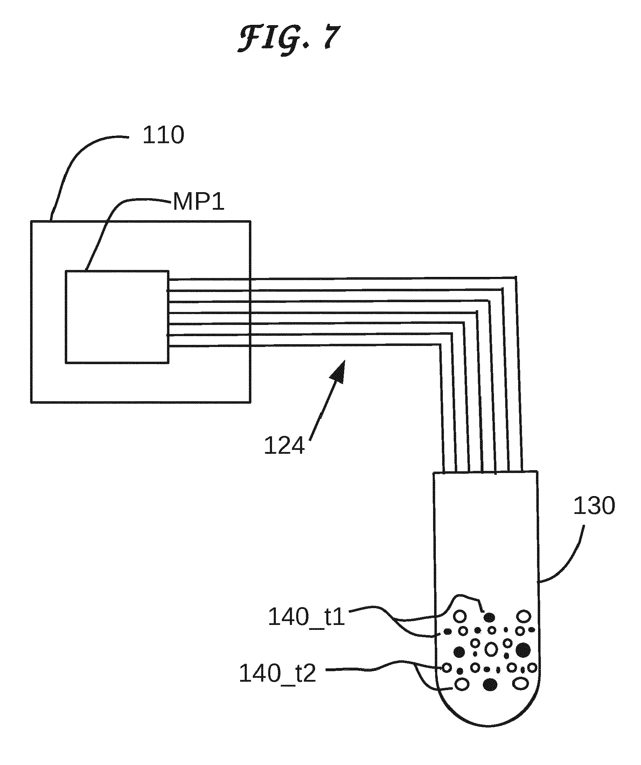

To physically achieve the above description, the controlling mechanism, in this case a microcontroller **MC1** residing with the battery/controlling electronics unit **BAT1** (FIG. 7) in sealed box **110,** is loaded with a computer program (or software), which is capable of executing automatic repetitive tasks following a programmed sequence which offers choices of values to be selected, the details of which are adjusted by a medical professional or by the patient himself, which determines a particular combination of active and passive electrodes to use, including the possibility to able to set the electric potential (voltage) at each electrodes of each type to use, also able to send this information by wires **124** to the stimulating unit **130.** Not all these options need to be available in a particular device. For example, a device using the invention could offer only 2 passive and 2 active electrodes and at a single electric potential (voltage) and still be within the scope of the invention. The correct sequence can be determined, for example, by the examination of an EKG (Electro Cardiogram) while varying the active electrodes of each type, their voltages and relative time sequence. Microprocessor **MP1,** located in box **110,** select which wires **124** to be connected to electric power and the voltage level as well, which may be different at each wire **124.** Each were **124** connects to one of the electrodes **140-t1** or **140-t2.** Each electrode type can be turned on or off (connected or disconnected from the electrical power) under the control of microprocessor **MP1.**

**Theory: the electric field lines.**

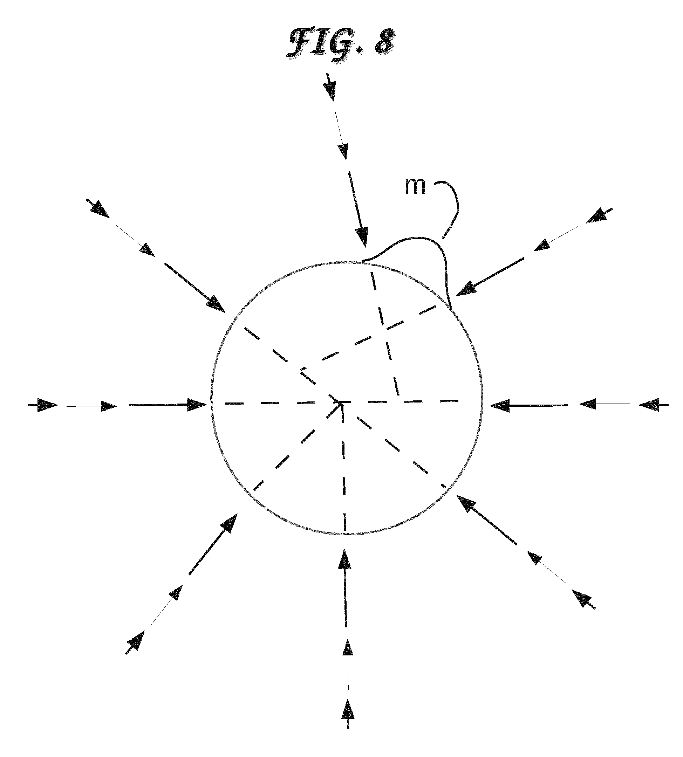

The solution to the problem of the best heart muscle contraction sequence is found in the theoretical analysis of electric current propagation within an electric field. As a side remark, this is similar to the motion of an object acted upon by the gravitational field of a planet, which is vertical towards the center of the planet, assuming a perfectly spherically symmetrical planet. All objects, unless prevented from falling by some means, do fall down in the direction of the center of the planet, as I would now, writing this text on a second floor. This attraction defines the straight vertical line. The earth gravitational field is composed of lines radially pointing to its center, as most of the field lines in FIG. 8. FIG. 8 also displays two gravitational field lines next to an exaggerated large mountain, which, due to its large mass tilts the gravitational field lines sideways towards the mountain. An actual large mountain does, surprisingly enough, minutely deflects the gravitational field from its "normal" direction towards the center of the earth, and in amounts that are detectable with modern equipment. This, of course, happens because the mountain attracts sideways.

Given that

$$\mathbf{F} \text{ (vector)} = q \times \mathbf{E} \text{ (vector)},$$

It follows that the force, and consequently the acceleration and then the motion of an electrically charged particle starting from rest is deflected by the electric field lines. The electric field can take more complex configurations than the gravitational field, because there are two types of electric charges (conventionally called positive and negative), while the gravitational field is due to only one type of gravitational charge (called mass, they only attract each other). FIG. 4 (a, b, c, d and e) displays five types of simple electric field configurations: Figures FIG. 4a and FIG. 4b display two cases of field lines that are simpler to calculate, of two electric charges, in fact the configuration normally seen in introductory physics books. The field lines keep deflecting the moving changes towards its direction, the actual path being a combination of the velocity and the deflection caused by the field lines. In other words, the field lines control the flow path of the injected current. From this it follows that to shape the electric field lines is the same as to lay down the "roads" where the current will travel whenever charges are set free in the region. This notion of shaping the field lines to determine the current path is seldom used only because in most electric circuits the current (electric charge) is forced to follow the wires, the coils, the transistors, etc., with no place for an externally imposed electric field to have any effect.

FIG. 4c shows a more complicated case with three charges. The reader is invited to observe the large change of the configuration of the field lines caused by the addition of this third charge, in particular the disappearance of the symmetry that is obvious in figures FIG. 4a and 4b. FIG. 4d and 4e display the effect of varying the value of the third charge. Again the reader is invited to ponder on the consequences of varying the values of the charges. Notice that both FIG. 4d and FIG. 4e are asymmetric, yet the shape of the field lines is vastly different between them!

The electric field lines are distinctively unequal, very different shapes. Not displayed is also their strengths, which is also distinct, left out to simplify the figure. FIG. 4 illustrates the point of our invention: a method and a means to conform the electric field lines to the desired 3-dimensional shape required for a most desirable heart squeezing sequence. In fact, using the piquita of our invention, it is possible to even create a 3-D electric field which causes a better heart squeezing sequence than the sequence that happens in a normal, healthy heart, because a normal, typical, healthy heart does not actually follow the best possible sequence! The reader is also asked to observe FIG. 1, which shows another variation of the piquita of our invention with a multiplicity of passive electrodes along the wire that leads to the electrodes. These extra passive electrodes adds to the potential handles with which to shape the electric field lines. Finally, the reader is also asked to remember that each of the passive electrodes may be made of supercapacitors, with the objective of increasing their effect on the field lines, which depend on the total charge at the electrodes, which becomes higher if the electrodes are made as supercapacitors.

Setting each small electrode at the surface of the piquita at a different electric potential (which causes a different electric charge Q on each electrode), a different electric field is set in its neighborhood. The cardiologist, or any other medical personnel, using a computer program to display the electric field created by any particular combination of voltages, will adjust the voltages at different electrodes and see, on the computer screen, the 3-D conformation of the electric field created by them. This is one problem of the class known as "inverse problems", a technical name given in mathematics for problems in which a particular cause is sought (a particular distribution of voltages on the surface of the piquita) for a particular 3-D electric field configuration over the heart muscles. Mathematicians have goose bumps when they are presented with an inverse problem, because they know that most inverse problems have no solution (no closed form solution, to be precise), nor does this one: it is not possible to solve these equations backwards. The solution of such an inverse problem is found by trial and error, adjusting a new charge distribution Q and noticing if the new electric field got closer to the desired one or farther away from it. From this, readjust the charges and observe the result again, and again, etc. Though this may seem a tedious solution, it is easier than working from scratch, because the hearts are approximately the same, and the pacemakers are implanted in approximately the same places, which means that the general type of solution needs to be found once and for all - then only smaller adjustments are necessary. In any case, if so desired the cardiologist can set all the active surface to be at the same electric potential (voltage), and set the passive electrodes at zero voltage, in which case the "improved" electric stimulator (pacemaker) would be working in the same way as prior art pacemakers. In practice, the inventors believe that even without individual adjustments, and only using the best average selection of surface distribution of electric potentials (voltages), there would be some improvement over existing electrical stimulators.

Current devices for heart pacemaking uses two and even three individual electrodes, for example, one electrode near the sino-atrial node (at the top of the right atrium), and one near the bottom of each ventricle (right and left). Multielectrode stimulators much enhance the performance of our invention, because they increase the number of available points over which there is control for adjusting the voltage V (or charge Q, which is the same thing), and also at much larger distances between them. More control is possible with the modern two- and three-stimulators using the technique known as Cardiac Resynchronization Therapy (CRT) than with the one single electrode at the top of the atrium.

**Introduction to the mathematical treatment of the problem of the best electric current distribution over the heart muscle.**

It is a well known result in electromagnetic theory that given a volume enclosed by an imaginary closed surface, any arbitrary time-dependent electromagnetic vector field obeying Maxwell's equations can be created adjusting the electric charge distribution at the surface that encloses the closed volume (see Reitz, Milford and Christy (1980), Jackson, (1975) or most any other introductory text in electromagnetic theory). This is valid for electromagnetic waves described by Maxwell's 2nd order differential equations. For the electrostatic case, Dirichlet's principle states that if a scalar function u(x) is a solution to the Poisson's equation

$$\mathrm{grad\ u} + f = 0\,,$$

on a domain $\Omega$ on $R^n$ ($R^3$ in our case),
with boundary condition u (x) = g (x),
then u (x) can be obtained as the minimizer of the Dirichlet's energy

$$E \{ u(x) \} = \text{Integral-on-}\Omega \{ dx [ (\tfrac{1}{2}) (\mathrm{grad\ }v)^{\wedge}2 - v * f ] \}$$

amongst all twice differentiable functions v(x) such that v(x) = g(x) on the specified domain. In our case u(x) is the electric potential V such that minus grad (V) is the electric field E:

$$\mathbf{E} = - \mathrm{grad}\ (V)$$

In the medical case, where the volume inside $\Omega$ encloses a heart or a brain, etc, so the surface $\Omega$ cannot be closed, the above statements are not applicable. Nevertheless, Lara's conjecture for incomplete boundaries states that a characteristic charge distribution exists on the surface that creates an electric field inside the surface that differs minimally from the desired value for small holes in $\Omega$. Consequently the Lara conjecture guarantees a reasonable solution for the medical case.

Dirichlet's problem is discussed in books dealing with electromagnetism because it is much related to the problems of interest in the field, yet it was initially developed out of its mathematical interest, and it is also discussed in many books in differential equations and potential theory.

This mathematical theory indicates that our invention works better with either a larger area supporting electrodes (which approaches a totally containing surface), as a planarium, and also with just a few small electrodes spread apart, as in the two- and three-electrodes of current heart pacemaking, anchored as they are, at the top of the right atrium and bottom of each ventricle, particularly if a number of passive electrodes are added along the wires leading to the active electrodes at the end of the wires.

DESCRIPTION AND OPERATION OF ALTERNATIVE EMBODIMENTS

Another embodiment of our invention is application to DBS (Deep Brain Stimulation). In this application the objective is to disrupt the anomalous neurons firings that cause the tremor characteristic of Parkinson's disease, or of what is known as essential tremor. One of the possible solutions is to place an electrode on a chosen target area in the brain then superimpose a current of frequency around 200 Hz on it. FIG. 7 shows a brain-type stimulator we call picafina, similar in structure to stimulators used today, with 4 rings at their distal extremity (Butson and McIntyre (2006)), but with the equivalent electrode described for the heart piquita: passive and active electrodes. The objective for the Deep Brain Stimulator (DBS) of our invention is to adjust the electric field in the vicinity of the picafina brain electric stimulator, to the shape of the particular target volume, which could be the sub-thalamic nucleus (STN), the globus pallidus internus (GPi) or any other volume defined by the neurosurgeon as the best for the particular patient. Much effort has been put on the solution of this problem, the solution of which has evaded the practitioners of the art for decades - see, for example, Butson and McIntyre (2006). It can be seen at Butson and McIntyre (2006) that the best solution they proposed is still a symmetric field. Such a symmetric field fail to offer a maximum electrical stimulation in any case, particularly when the electric stimulator happens to have been implanted off-center. As discussed by Butson and McIntyre (2006), this is, in fact, a most common occurrence, due to the small size of the target volumes and their location deep in the base of the brain (for DBS), which is also not directly observed by the surgeon, which inserts the electric stimulator through a one-cm diameter hole drilled at the top of the skull, from where she tries to guide the stimulator tip to the desired target. Our invention allows for more control of the electric field around the stimulator, which in turn, allows for better clinical results. More modern stimulators, e.g. the ones introduced by Sapiens Neuro and by Rubert Martens et a/., "Spatial steering of deep brain stimulation volumes using a novel lead design" Clin. Neurophys. Vol 122 pg 558-566 (2011) are capable of creating an asymmetric electric charge distribution in the target area, but fail to decouple the control of the electric field from the injection of the electric charges, therefore failing to maximize the results. Sapiens Neuro brain stimulator is capable of injecting electric charges towards one arc, but not capable of keeping a desired electric field within the target volume to keep the injected charges in the desired volume as our invention does. Moreover, Sapiens Neuro stimulator fails to apply a driving force on the electric charges after they are injected into the target volume, which our invention does.

The electrodes for DBS can be of different size, of different shapes and also randomly distributed on the surface of the supporting structure or picafina, or they can be of uniform size and shape, perhaps to decrease manufacturing cost, for example, or to simplify the internal wiring, or any other reason, and they can be also geometrically arranged instead of randomly distributed on the surface. Given the small size of the electrodes, random shape of them is of smaller effect than their numbers, while the use of the two types of electrodes, active or type-1 electrodes and passive or type-2 electrodes are of major importance, given that the latter only change the electric field shape around the stimulator device.

The reader will notice that the DBS application is a natural adaptation of all that is described for the heart pacemaker, yet the DBS is less likely to need time control because there is no sequential muscular contraction, so it is simpler to program and to use than the heart piquita. Yet, situations may arise where time delays between different electrodes may be useful to cause the stimulation to reach some desirable target locations. A multiplicity of electrodes, of variable shapes and sizes, each associated with a unique wire, which is used to select which electrode is turned on, which electrode is turned off, both for type-1 (active) and type-2 (passive). Likewise for the heart pacemaker, the DBS incarnation uses two types of electrodes: a first type, or active type, capable of injecting a current, and a second type, or passive type, which is insulated, not capable of injecting any current (though always there is a small leak current due to insulator imperfections), but which is much useful for creating the vector field around the electrode, which, in turn, determine the 3-D path for the injected current.

Another possible application for the invention is for appetite control. In this application there are at least two possibilities: electrical stimulation on the stomach, and brain stimulation at the locations which are known to control the appetite or at the nerves that carry the information from/to the brain. In the former case the added electrical stimulation may be turned on before a meal, and the electrodes are selected to affect the neurons that send information to the brain regarding the current amount of food in the stomach, which in turn modulate the appetite. If the stimulation is capable to fool the brain, the individual will feel a decreased urge for food, eat less, and lose weight on the long run. The first application has been used in humans already. The second case, brain stimulation to control the appetite has been only used in animals so far, and with success. For stomach stimulation the shape of the stimulator should be a flat shape to conform to the curvature of the stomach and its enervations, a variation of what we call planarium. For direct brain control it may be similar in shape to the DBS.

Another possible application is for cortical brain stimulation, in which case the stimulator has a flat shape to adjust to the cortical application. We call planarium the flat, or sheet-type stimulator.

Another possible application is for pain control, an improvement of a known device known as TENS (Transcutaneous Electrical Neural Stimulation). In this application the objective is to control superficial pain, as skin pain, and it has used for deeper pain too, as muscle pain. The area in question is in this case surrounded by electrodes attached to the skin, from which a current flows (here it is really an area, the surface area of the skin in question, not what the neurologists call area in the brain, which is a volume). A similar method is in use already, but with larger electrodes, which cannot control the depth and direction of the electric current that is injected, while the passive electrodes of our invention is useful to control the direction and depth of electric current for the same reasons as for the heart muscle, only that in this case the timing is of less importance than with the heart. Also the existing devices use large electrodes, which did not allow for precise control of the point of electric current injection. In this case our invention discloses a large number of small electrodes which are on the surface of the applied patch. Likewise the heart pacemaker, these small electrodes may be numbered or otherwise identifiable by any means, and individually activated by their dedicated wires which is under control of the controlling electronics, are of two types (type-1, or active, and type-2, or passive), and can likewise be turned on at any of a plurality of voltages/currents or off (zero voltage/current). With a wise selection of the active electrodes, it is possible for the medical practitioner to ameliorate the pain felt by the patient in a more effective way than currently used TENS devices because our invention allows for more control of the electric current injected in the patient.

Another variation is the same TENS device described above but with one (or a few) wires used to set a fixed electric potential (voltage), which is then connected to particular electrodes using digital switches or even manual switches. The electrodes may be of either type: active or passive.

Another variation is the use of the supercapacitor-type of passive electrodes **140_t2** on chochlear implants. In this case the passive electrodes **140_t2** can be used to direct the current in the direction of the single neuron towards which it is intended to propagate. Current cochlear implants suffer from a spreading effect, which is that the injected current spreads sideways, eventually activating several neurons, instead of just one, and therefore conveying to the brain the impression of more than one frequency, instead of just the one intended frequency.

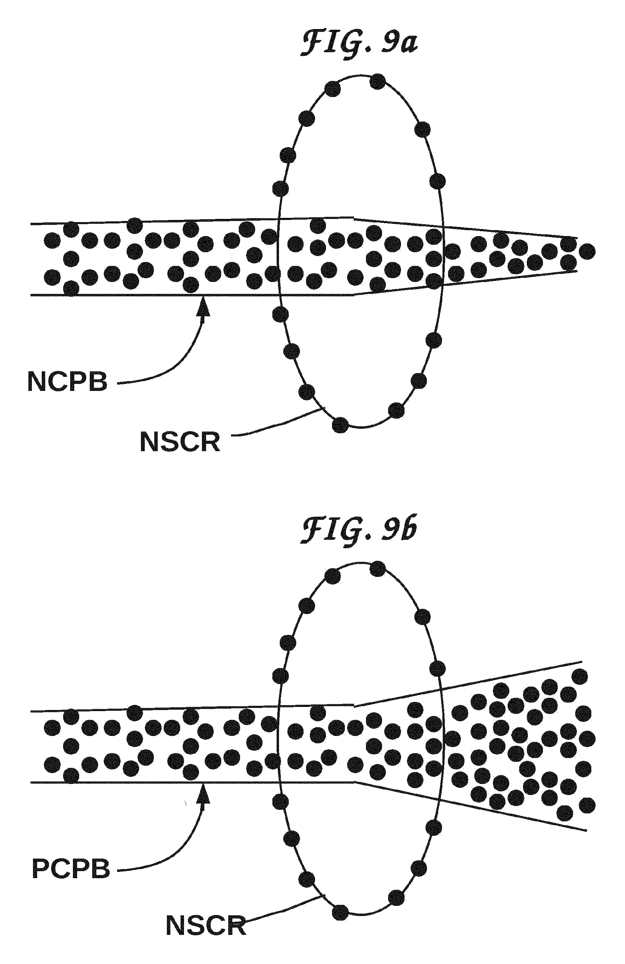

Another variation is the use of the supercapacitors on a ring support **SCR** which is coaxial with a charged particle beam as shown in FIG. 9. In FIG. 9 **NCPB** represents a negative charged particle beam, while a **PCPB** represents a positive charged particle beam, and **NSCR** represents a negatively charged supercapacitor ring, while **PSCR** represents a positively charged supercapacitor ring. FIG. 9a shows the focusing, or squeezing configuration, while FIG. 9b shows the defocusing, or spreading configuration. For beam focusing the supercapacitor ring **SCR** is charged with charges of the same polarity as the beam polarity (both negative in 9a), while for defocusing the supercapacitor ring **SCR** is charged with charges of opposite polarity as the beam polarity (say positive beam and negative charged supercapacitor in 9b).

CONCLUSION, RAMIFICATIONS, AND SCOPE OF INVENTION

The individual electrodes, which in the main embodiment are randomly spread on the supporting structure (piquita), and are of various shapes and sizes, can be all of the same shape and/or same size, and/or can be arranged on an orderly arrangement too. Cost and other factors could determine a simpler regular electrode arrangement. More orderly arrangements of the electrodes than the arrangement disclosed in the main embodiment, which provides maximal advantage, are still in the scope of the invention. For example, it is possible to control the vector injected electric current (magnitude and direction) with circular electrodes (of either type, active and/or passive ones) that are of *different sizes* and randomly distributed on the surface of the piquita. It is also possible to control the vector injected electric current with circular electrodes (of either type), that are of the *same size* and randomly distributed on the surface of the piquita. Or it is also possible to control the injected electric current vector with circular electrodes that are of the *same shape and size* and orderly distributed on the surface of the piquita, this being the most symmetric electrode arrangement of all. The difference between these options is simply the degree of possible variations and fine control on the vector current, and the choice between each option is based on a cost / benefit analysis, all being still within the scope of our invention.

Persons acquainted with the art of symmetry will recognize that very small electrodes with small spacing between each can simulate larger electrodes of variable shape and sizes, as particular sets of smaller electrodes can approximately create the shape of a larger electrode of any arbitrary shape. Cost and programming time may dictate one type of another of electrode, and their size and placement, while these variations are still covered in the scope of the invention.

The relative distribution of the electrodes of type-1 and type-2 (current injecting electrodes and electric field shaping electrodes, or magnitude and direction determining electrodes) is random in the main embodiment of this invention, but it is possible to alternate electrodes from type-1 to type-2, then type-1 again, etc., when the electrodes are of the same size and orderly distributed on the surface of the stimulating piquita, picafina, planarium and their variations.

Another way to see the control of the paths of the current in the heart, or the extent of electrical stimulation in brain DBS, etc., is to look at the active electrodes determining the magnitude (and also the direction in a limited way too, because the active electrodes also contribute to the electric field vector though for a very short time) and the passive electrodes determining the direction and speed only of the current injected by the former, active electrodes. In this view one considers the stimulating current as a vector which is directed by the electric field lines.

Other options are possible for the marker **140-tm** that indicates the angular position of the piquita with respect to the body in which it is inserted. For example, all the electrodes may have enough X-ray opacity to show in the fluoroscopic images taken during the heart pacemaker implantation. Or one or more or the anchoring arms **131** may be smaller (or larger), or each anchoring arm may be of a different length and/or diameter, to allow their identification.

The main embodiment for heart stimulation uses a simple version of stimulation, which is fixed and inflexible, of the type of the old heart pacemakers. It is possible to have stimulation on demand too, as many current pacemakers have, which is based, for example, on activating the stimulation only when the natural pacemaker becomes insufficient, or stops, or becomes erratic. This is called stimulation on demand, easily incorporated in our invention that already contains a microprocessor capable of implementing such decisions. Such extensions are part of the current art of heart pacemakers and may or may not be incorporated in our invention. Our invention is independent of stimulation on demand.

Following lawyer's practice I need to reluctantly add that one skilled in the relevant art, however, will readily recognize that the invention can be practiced without one or more of the specific details, or with other methods, etc. In other instances, well known structures or operations are not shown in detail to avoid obscuring the features of the invention. For example, the details of the wiring can be realized in several different ways, as coiled wires, as printed circuit wires, etc., many or most of which are compatible with the invention, and therefore the details of these, and other details are not included in this patent disclosure.

REFERENCES

Butson and McIntyre (2006). Christopher R. Butson and Cameron C. McIntyre "Role of electrode design on the volume of tissue activated during deep brain stimulation" Journal of Neural Engineering, vol. 3, pgs. 1-8 (2006)

Chong Il Lee and Sergio Lara Pereira Monteiro (2011) "Method and means to address and make use of multiple electrodes for measurements and electrical stimulation in neurons and other cells including brain and heart" US patent application number 13 / 053,137 , March 21, 2011, not yet published.

Chong Il Lee (2010) "Method and means for connecting a large number of electrodes to a measuring device" US patent application number 2010-0079156, published April 1, 2010

Chong Il Lee and Sergio Lara Pereira Monteiro (2010) "Method and means for connecting and controlling a large number of contacts for electrical cell stimulation in living organisms" U.S. patent application number 2010-0082076, published April 1st, 2010.

Colleen Clancy and Yang Xiang "Wrapped around the heart" News and Views, Nature 507, 43, (6 March 2014), attached.

DIRICHLET - http://en.wikipedia.org/wiki/Dirichlet principle

JamilleHetke_Kipke_Pellinen_Anderson_ModularMultichannelMicroelectodeArrayEtc_USPTO                    -Pat-Publ-US2007-0123765_070531

Jackson (1975) Jackson "Classical Electrodynamics" Wiley.

Lizhi Xu et al. "3D multifunctional integumentary membranes for spatiotemporal cardiac measurements and stimulation across the entire epicardium", Nature Communications DOI:10.1038/ncomms4329, attached.

Medtronic (n/d) Medtronic website with info on DBS leads. http://professional.medtronic.com/pt/neuro/dbs-md/prod/dbs-lead-model-3387/index.htm http://professional.medtronic.com/pt/neuro/dbs-pd/prod/dbs-lead-model-3391/index.htm

Pierre Martin "Une membrane artificielle pour surveiller le coeur" La Recherche no. 487 page 20, 1o Mai 2014, attached.

Dennis T. T. Plachta et al. "Blood pressure control with selective vagal nerve stimulation and minimal side effects" J. Neural Eng. 11, 036011 (2014).

Reitz, Milford & Christy (1980), John Reitz, Frederick Milford, Robert Christy "Foundations of Electromagnetic Theory" 3rd edition, 1980.

Thaler (2003) Malcolm S. Thaler "The Only EKG Book You'll Ever Need", Lippincott Williams & Wildins, 4th ed. (2003).

**Claims**

1. An implantable electrical stimulating device comprising:

    an electric energy storage unit **BAT1**;

a first controlling electronics part of **MP1;**

a second controlling electronics part of **MP1;**

a piquita supporting structure **132** comprising a proximal extremity, a distal extremity, an inner lumen with an outer surface;

a plurality of electrodes **140** comprising at least one electrode belonging to a group of active electrodes **140_t1** and/or to a group of passive electrodes **140_t2;**

wherein the active electrodes **140_t1** are configured to inject electric current into the body cells surrounding the piquita supporting structure **132** and;

wherein the passive electrodes **140_t2** are configured to project electric fields into the body cells surrounding the piquita supporting structure **132** while configured not to inject electric currents into the body cells surrounding the piquita supporting structure **132;**

wherein the first controlling electronics part of **MP1** comprises first electronic circuits to select a subset of the plurality of electrodes **140** to be operational;

wherein the second controlling electronics part of **MP1** comprises second electronic circuits to implement the selection from the first controlling electronics part of **MP1;**

wherein the electrically insulating layer on the passive electrodes **140_t2** acts as an insulator for DC current or low frequency cardiac signals, a opposed to the insulating layer to create a capacitor for capacitive coupling of the AC current;

wherein the electrical field lines projected by the passive electrodes **140_t2** direct the path of moving electric charge in the body cells where the electric field lines are located;

wherein the passive electrodes **140_t2** are configured to create an electric vector field in the body cells surrounding the piquita supporting structure **132,** the electric vector field **characterized by** a magnitude and a direction, wherein the direction determines a plurality of field lines, along which the electric current is injected., wherein the improvement over prior art consists in using supercapacitors for the passive electrodes **140_t2.**

2. The implantable electric stimulating device of claim **1,** further comprising an external programming unit, configured to provide instructions to the first controlling electronics part of **MP1** and to the second controlling electronics part of **MP1,** using wireless transmission, whereby a medical practitioner can adjust operation of the implantable electrical stimulating device.

3. The implantable electric stimulating device of claim 1, wherein the created electric vector field created by the at least one electrode of the group of passive electrodes **140_t2** is configured to force the path of the electric current injected by the active electrodes **140_t1** to move along a path and at a speed influenced by the created electric vector field lines.

4. The implantable electric stimulating device of claim 3, wherein the at least one electrode belonging to the group of passive electrodes **140_t2** are covered by an electrically insulating layer to prevent electric current from being injected into the body cells.

5. The implantable electric stimulating device **132** of claim **1,** further comprising a first binary digital addressing means part of **MP1** to select each of the plurality of the electrodes on the body of the piquita supporting structure **132,** wherein the first controlling electronics part of **MP1** is configured to cause the second controlling electronics part of **MP1** to select a first subset of the at least one electrode of a group of active electrodes **140_t1** to be operational and a second subset of the at least one electrode of a group of passive electrodes **140_t2** to be operational.

6. The implantable electric stimulating device **132** of claim **1,** wherein the first and/or the second controlling electronics **MP1** is/are configured to select a plurality of electric voltage or electric current values, wherein each of the at least one active electrode **140_t1** and at least one passive electrode **140_t2** has a voltage/current value each passive electrode **140_t2** and each active electrode **140_t1** a different voltage level a different current level same voltage level or the same current level.

7. The implantable electric stimulating device of claim **6,** further comprising a plurality of voltage wires or current wires **124,** each of the voltage wires **124** or current wires **124** to convey one voltage value and/or one current value.

8. The implantable electric stimulating device of claim **7,** further comprising a second binary digital addressing means to select each voltage or current for electrical connection to a subset of the at least one of the group of active electrodes **140_t1** and/or the at least one of the group of passive electrodes **140_t2.**

9. The implantable electric stimulating device **132** of claim **1,** further comprising at least one digital serial bus to transfer digital binary information.

10. The implantable electric stimulating device of claim **9,** wherein the digital serial bus is a digital part of a USB bus.

11. The implantable electric stimulating device of claim **1,** wherein the piquita supporting structure **132** is adapted to be anchored in a heart of an animal, including a human animal.

12. The implantable electric stimulating device of claim **1,** wherein the first controlling electronics includes a microprocessor **MP1.**

13. The implantable electric stimulating device of claim **1,** wherein the active electrodes **140 _t1** and the passive electrodes **140_t2** are randomly distributed on the body of the piquita supporting structure **132.**

14. The implantable electric stimulating device of claim 1, wherein the at least one electrode belonging to the group of active electrodes **140_t1** and the at least one electrode belonging to the group of passive electrodes **140_t2** are symmetrically distributed on the body of the piquita supporting structure **132.**

15. A method to activate at least one electrode **140** in a multichannel electrode array having a plurality of active electrodes **140_t1** and a plurality of passive electrodes **140_t2,** the method comprising:

    applying a first voltage on at least one of the plurality of passive electrodes **140_t2,** wherein the at least one passive electrode **140_t2** is configured to create an electric vector field in body cells surrounding the at least one passive electrode **140_t2;**
    applying a second voltage on at least one of the plurality of active electrodes **140_t1,** wherein the at least one active electrode **140_t1** is configured to inject an electric charge in the body cells surrounding the at least one active electrode **140_t1;**
    wherein the electrically insulating layer on the passive electrodes **140_t2** acts as an insulator for DC current or low frequency cardiac signals, as opposed to the insulating layer to create a capacitor for capacitive coupling of the AC current;
    wherein the electrical field lines projected by the passive electrodes **140_t2** direct the path of moving electric charges in the body cells where the electric field lines are located;
    wherein the electric vector field is configured to control the direction and speed of the electric charges injected in the body cells.
    wherein the inventive method consists of using supercapacitors for the construction of the passive electrodes **140_t2.**

16. The method according to claim **15,** further comprising simultaneously activating at least two of the plurality of passive electrodes **140_t2** using a different level of voltage on each of the two of the plurality of passive electrodes **140_t2;** wherein at least two electrodes of the plurality of passive electrodes **140_t2** further increases the control of the shape of the electric vector field as compared with a single electrode of the plurality of passive electrodes **140_t2.**

17. The method according to claim **15,** wherein the plurality of passive electrodes **140_t2** uses a monopolar configuration having a remote ground.

18. The method according to claim **15,** wherein a first subset of electrodes of the multichannel electrode array are configured to be of a positive polarity and a second subset of electrodes are configured to be of a negative polarity; wherein the first subset of electrodes and second subset of electrodes comprise different electrodes.

# FIG. 1

# FIG. 2

1 mm

# FIG. 3

## FIG. 4a

## FIG. 4b

+q     -q

+q     +q

## FIG. 4c

-q/2

+q

+q

*FIG. 4d*

−q/10

+q

+q

*FIG. 4e*

−2.5q

+q

+q

# FIG. 5

FIG. 6a

FIG. 6b

# FIG. 6c

*FIG. 7*

110

MP1

124

130

140_t1

140_t2

FIG. 8

m

## FIG. 9a

NCPB

NSCR

## FIG. 9b

PCPB

NSCR

**EUROPEAN SEARCH REPORT**

Application Number

EP 15 16 1397

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | EP 2 522 389 A2 (LARA PEREIRA MONTEIRO SERGIO [US]; LEE CHONG II [US]) 14 November 2012 (2012-11-14) * paragraph [0039] - paragraph [0050] * * paragraph [0067] - paragraph [0077]; claim 1; figure 2 * | 1-18 | INV. A61N1/05 A61N1/40 |
| Y | US 2014/029162 A1 (HUR JAE-HYUN [KR] ET AL) 30 January 2014 (2014-01-30) * paragraph [0082] - paragraph [0087] * * paragraph [0100] - paragraph [0103] * * paragraph [0195] - paragraph [0196] * | 1-18 | |
| A | US 2009/088811 A1 (WULFMAN DAVID R [US]) 2 April 2009 (2009-04-02) * paragraph [0072] - paragraph [0080] * * paragraph [0093] - paragraph [0098]; figure 2 * | 1-18 | |
| A | US 2013/317580 A1 (SIMON BRUCE J [US] ET AL) 28 November 2013 (2013-11-28) * paragraph [0190] - paragraph [0191] * * paragraph [0232] - paragraph [0235] * | 1-18 | TECHNICAL FIELDS SEARCHED (IPC) A61N |
| A | US 5 312 439 A (LOEB GERALD E [CA]) 17 May 1994 (1994-05-17) * column 1, line 44 - line 55 * * column 3, line 42 - line 49 * * column 5, line 51 - column 6, line 2; figure 1 * | 1-18 | |

The present search report has been drawn up for all claims

| Place of search | Date of completion of the search | Examiner |
|---|---|---|
| Munich | 15 December 2015 | Sigurd, Karin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 16 1397

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-2015

| Patent document cited in search report | | Publication date | Patent family member(s) | | Publication date |
|---|---|---|---|---|---|
| EP 2522389 | A2 | 14-11-2012 | EP | 2522389 A2 | 14-11-2012 |
| | | | US | 2012289823 A1 | 15-11-2012 |
| US 2014029162 | A1 | 30-01-2014 | CN | 103578786 A | 12-02-2014 |
| | | | US | 2014029162 A1 | 30-01-2014 |
| US 2009088811 | A1 | 02-04-2009 | EP | 2211979 A1 | 04-08-2010 |
| | | | JP | 2010540073 A | 24-12-2010 |
| | | | US | 2009088811 A1 | 02-04-2009 |
| | | | US | 2009088812 A1 | 02-04-2009 |
| | | | WO | 2009042063 A1 | 02-04-2009 |
| US 2013317580 | A1 | 28-11-2013 | NONE | | |
| US 5312439 | A | 17-05-1994 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62027116 B
- US 8954145 B
- EP 20120167688 A
- EP 2522389 A3
- US 8335551 B
- US 8538516 B
- US 8565868 B
- US 13053137 B
- US 20100079156 A
- US 20100082076 A
- US 20070123765070531 A

### Non-patent literature cited in the description

- **DENNIS T. T. PLACHTA et al.** *Plachta,* 2014
- **RUBERT MARTENS.** Spatial steering of deep brain stimulation volumes using a novel lead design. *Clin. Neurophys.,* 2011, vol. 122, 558-566
- **CHRISTOPHER R. BUTSON ; CAMERON C. MCINTYRE.** Role of electrode design on the volume of tissue activated during deep brain stimulation. *Journal of Neural Engineering,* 2006, vol. 3, 1-8
- **COLLEEN CLANCY ; YANG XIANG.** Wrapped around the heart. *News and Views, Nature,* 06 March 2014, vol. 507, 43
- **JACKSON.** Classical Electrodynamics. Wiley, 1975
- **LIZHI XU et al.** 3D multifunctional integumentary membranes for spatiotemporal cardiac measurements and stimulation across the entire epicardium. *Nature Communications*
- **PIERRE MARTIN.** Une membrane artificielle pour surveiller le coeur. *La Recherche no. 487,* May 2014, 20
- **DENNIS T. T. PLACHTA et al.** Blood pressure control with selective vagal nerve stimulation and minimal side effects. *J. Neural Eng.,* 2014, vol. 11, 036011
- **JOHN REITZ ; FREDERICK MILFORD ; ROBERT CHRISTY.** Foundations of Electromagnetic Theory. 1980
- **MALCOLM S. THALER.** The Only EKG Book You'll Ever Need. Lippincott Williams & Wildins, 2003